# EUROPÄISCHE PATENTANMELDUNG

(11) **EP 3 685 810 A1**
(43) Veröffentlichungstag der Anmeldung: **29.07.2020**
(21) Anmeldenummer: 19153229.0
(22) Anmeldetag: 23.01.2019
(51) Int. Cl.: A61F 7/08, G16H 40/67

(54) **MOBILE VORRICHTUNG ZUR BEHANDLUNG VON JUCKREIZ MIT SCHNITTSTELLE**

(71) Anmelder: Dermapharm AG, 82031 Grünwald (DE)
(72) Erfinder: Bünger von Wurmb, Daniel, 41063 Mönchengladbach (DE)
(74) Vertreter: Hertin und Partner Rechts- und Patentanwälte PartG mbB

(57) **Zusammenfassung**

Die Erfindung betrifft bevorzugt eine Vorrichtung zur hyperthermischen Behandlung von Juckreiz und/oder Herpeserkrankungen umfassend eine Regelvorrichtung auf welcher mindestens zwei Behandlungsprogramme gespeichert vorliegen sowie eine Interface zur Auswahl eines der mindestens zwei Behandlungsprogramme durch ein Mobilgerät, sodass nach Betätigung eines Bedienelementes die Regelvorrichtung gemäß des ausgewählten Behandlungsprogrammes eine Behandlungsfläche auf eine Behandlungstemperatur zwischen 40° - 65°C erhitzt und diese für eine Behandlungsdauer zwischen 1 und 12 Sekunden hält.

## Beschreibung

Die Erfindung betrifft bevorzugt eine mobile Vorrichtung zur hyperthermischen Behandlung von Juckreiz und/oder Herpeserkrankungen umfassend eine Regelvorrichtung auf welcher mindestens zwei Behandlungsprogramme gespeichert vorliegen sowie ein Interface zur Auswahl eines der mindestens zwei Behandlungsprogramme durch ein Mobilgerät, sodass nach Betätigung eines Bedienelementes die Regelvorrichtung gemäß des ausgewählten Behandlungsprogrammes eine Behandlungsfläche auf eine Behandlungstemperatur zwischen 40° - 65°C erhitzt und diese für eine Behandlungsdauer zwischen 1 und 12 Sekunden hält.

### Hintergrund und Stand der Technik

Juckreiz (Pruritus) ist eine subjektiv unangenehme, auf die Haut oder Schleimhaut bezogene Sinneswahrnehmung. Sie kann lokal begrenzt sein oder aber den ganzen Körper betreffen. Häufig geht Juckreiz mit einem brennenden, stechenden oder kribbelnden Gefühl einher, welches die betroffene Person oftmals durch Kratzen, Scheuern, Rubbeln, Drücken, Kneten oder Reiben versucht zu lindern. Daher kommt es bei Juckreiz gehäuft zu weiteren pathologischen Erscheinung der Haut wie Kratzspuren, offene Wunden, Krustenbildung und Hautinfektionen. Die Fachwelt geht davon aus, dass Juckreiz über Schmerzrezeptoren der Haut vermittelt wird und über das vegetative Nervensystem zum Gehirn geleitet wird. Die Ursachen von Juckreiz können sehr vielfältig sein. Neben trockener Haut, fehlender Feuchtigkeitszufuhr oder Allergien kann Juckreiz auch durch äußere Einwirkungen und Hautreizungen, wie z.B. durch Stiche von Mücken oder nach Kontakt mit Nesseltieren entstehen. Juckreiz kann eine Reaktion auf chemische, mechanische oder thermische Reize sein. Es können als Folge der äußerlichen Reizung, wie z.B. durch Einwirkungen chemischer Substanzen, z.B. Histamin (Mückenstich), Apamin (Bienenstich), durch allergische Immunreaktion, durch Druck oder Reibung oder auch durch Wärme oder Sonnenbestrahlung, Quaddeln, Urtikaria und andere mit Juckreiz verbundene Hautreaktionen hervorgerufen werden. Aus medizinischer Sicht spannen die Ursachen oder Grunderkrankungen, welche zu Juckreiz führen ein breites Spektrum dermatologischer und internistischer Erkrankungen auf.

Zur medikamentösen Behandlung von den Symptomen des Juckreizes sind eine Reihe von Medikamenten oder kosmetischen Produkten bekannt. So werden ätherische Öle insbesondere umfassend Menthol, Thymol oder Campher verwandt, um kurzfristig eine Kühlung zu erzeugen. Auch Hautpflegemittel wie beispielsweise Cremen oder Lotion können durch eine Erhöhung des Feuchtigkeitsgehaltes der Haut eine schmerzlindernde Wirkung entfalten. Darüber hinaus stellen Antihistaminika hilfreiche Therapiemöglichkeiten dar, welche beispielsweise die Gabe von Dimetindenmaleat oder Mepyramin umfassen. Weiter Medikamente betreffen topische Glucocorticoide, Anästhetika, Zinksalben, Calcineurinhemmer oder Capsaicin. Zur Behandlung von Wespen oder Bienenstichen werden weiterhin die Einstichstellen auch mit Salmiakgeist behandelt, welches jedoch nur zu einer kurzfristigen Linderung des Juckreizes führt und auch die Schwellung nur im geringen Maße zurückgehen lässt.

Aus dem Stand der Technik ist es jedoch auch bekannt durch Einbringung einer Wärmemenge in den Einstich von Insekten das Auslösen eines Juckreizes zu mindern. Eine Vorrichtung für eine lokale, thermische Behandlung insbesondere von Mückenstichen wird in der EP 1231875 B1 beschrieben. Die Vorrichtung weist eine Heizplatte mit einer Größe von ca. 0,2 cm² auf, welche auf eine Temperatur zwischen 50°C und 65°C gebracht wird, während die Heizplatte den Insektenstich kontaktiert. Durch diese hyperthermische Behandlung lässt sich der Juckreiz nachhaltig lindern. Zum einen resultiert die Wärmeapplikation in einem Abbau der thermolabilen Giftstoffe der Insekten, welche den Juckreiz auslösen. Zum anderen führt die Wärmeübertragung zu einer Überlagerung des Juckreizes durch andere temperaturabhängige Hautempfindungen. Durch derartige Behandlungen können somit weiterhin sekundäre Schädigungen der Haut, beispielsweise eine Entzündung des Insektenstiches durch Kratzen, wirksam vermieden werden. Somit wird durch die hyperthermische Behandlung auch das mit einem Insektenstich einhergehende Auftreten von Quaddeln wirksam reduziert.

Die Anwendungsmöglichkeiten einer hyperthermischen Behandlung erstrecken sich weiterhin auf Herpeserkrankungen.

Herpes ist allgemein als akute, primäre oder sekundäre Viruserkrankung der Haut und der Schleimhäute durch Infektion durch das Herpes-Virus bekannt. Sie tritt meist als örtlich begrenzt gruppiert stehende Bläschen vor allem im Gesicht (Herpes *facialis*), an den Wangen (Herpes *buccalis*), der Nase (Herpes *nasalis*) sowie an den Geschlechtsorganen (Herpes *genitalis*) auf. Die Erstinfektion erfolgt oft im früheren Kindesalter und ohne erkannt zu werden, führt aber zur Bildung von Antikörpern und oft zu Maskierung des Virus und Absiedlung in Körpergeweben, die bei den Virusträgern bei Schwächezuständen des Immunsystems zu Rückfällen führt. Zur lindernden Behandlung von Schmerzen und Juckreiz bei rezidivierendem Herpes *labialis* werden Lippenherpescremes z. B. mit dem Wirkstoff Aciclovir benutzt. Diese Cremes sollen bereits bei den ersten Anzeichen einer Herpeserkrankung (Brennen, Jucken, Spannungsgefühl und Rötung) angewendet werden, da sie das Wachstum bestimmter Viren hemmen (Virostatikum, Virus-DNS-Polymerasehemmstoff). Die Behandlungsdauer beträgt im Allgemeinen 5 Tage. Nachteilig bei derartigen Cremes ist es, dass mitunter Nebenwirkungen und Unverträglichkeiten auftreten oder die erhofften Wirkungen ganz oder teilweise ausbleiben.

Aus der DE 102005002946 A1 ist eine Vorrichtung zur hyperthermischen Behandlung von Herpeserkrankungen bekannt. Die Vorrichtung umfasst eine Heizplatte mit einer bevorzugten Größe von 20 mm², welche für eine Behandlungsdauer von vorzugsweise 10 - 15 sec auf 49 °C - 53 °C erhitzt wird. Während der Behandlungszeit kontaktiert die Heizplatte die betroffene Hautpartie der Lippen, beispielsweise die gerötete Stelle oder aber die Position an der sich bereits Bläschen gebildet haben. Die Hitzeapplikation führt zum einen zu einer Eindämmung der Vermehrung der ursächlichen Krankheitserreger durch eine neutralisierende Wirkung auf die Herpes-simplex-Viren. Zum anderen kommt es durch die kurzzeitige Wärmebehandlung zu einer Überlagerung des Juckreizes der Herpeserkrankung durch die Stimulation temperatursensitiver Nerven. Die Vorrichtung zeichnet sich somit durch eine Reduktion der Symptome der Herpeserkrankung wie z.B. Brennen, dem Auftreten von Schwellungen, einer Rötung oder eines Juckreizes aus.

Die aus dem Stand der Technik bekannten Vorrichtungen zur hyperthermischen Behandlung zeichnen sich durch vielfältige Einsatzmöglichkeiten zur Linderung der Symptome von Insektenstichen, Herpeserkrankungen, Quallenstichen oder anderen mit Juckreiz einhergehenden Erkrankungen aus. Die Vorrichtungen weisen jedoch auch Nachteile auf.

In Ausnahmefällen kann es beispielsweise zu einer Überschreitung der gewünschten Behandlungstemperatur kommen.

Im Stand der Technik ist es daher bekannt die Behandlungstemperatur mithilfe von Temperatursensoren zu überwachen. Durch Beschädigungen des Gerätes, beispielsweise durch das Eintreten von Feuchtigkeit, kann es jedoch zu einer Beeinträchtigung des Regelkreislaufes der Überwachungselektronik kommen. Dies ist insbesondere der Fall, wenn die Überwachung der Behandlungstemperatur in den regulären Steuerkreis eingebunden ist. In diesem Fall ist es nicht auszuschließen, dass es zu einer Überhöhung der Temperatur über die gewünschte Behandlungstemperatur hinaus kommt. In Abhängigkeit von der Kontaktposition der Heizplatte bzw. Behandlungsfläche treten hierdurch unerwünschte Nebenwirkungen auf. Selbst bei einer kurzzeitigen Temperaturerhöhung von über 65°C kann es zu nachhaltigen Schädigungen der betreffenden Hautpartien kommen. Dies ist insbesondere der Fall für empfindliche Hautpartien, wie beispielsweise die Lippen während einer Herpesbehandlung oder aber auch dünnere Hautpartien, an welchen Insektenstiche vorliegen.

Aus der US 2007/0049998 A1 ist ein Gerät zur hyperthermischen Behandlung von Hautbeschwerden bekannt, welches eine Behandlungsfläche über ein temperaturgeregeltes Heizelement auf Temperaturen von 38 °C - 67 °C für eine Dauer von mindestens 5 Sekunden, typischerweise jedoch über einen längeren Zeitraum, aufheizt und zur Sicherung gegen Überhitzen eine Schmelzsicherung verwendet. Diese Art der Absicherung gegen Überhitzen hat den Nachteil, dass bei einem Auslösen durch Überhitzen ein Austausch der Sicherung erfolgen muss. Außerdem ist kein redundanter Sicherheitsmechanismus vorhanden und bei Versagen der Schmelzsicherung droht eine Überhitzung der Behandlungsfläche über einen längeren Zeitraum. Des Weiteren werden Temperaturen ab 60 °C oder darüber hinaus, besonders über einen längeren Zeitraum von einigen Sekunden oder länger, als sehr unangenehm empfunden und können zu Hautschädigungen führen. Dies kann mindestens dazu führen, dass der Behandlungserfolg gefährdet wird, weil Behandlungen aufgrund eines unangenehmen Hautgefühls durch die hohen Temperaturen frühzeitig abgebrochen werden und somit der Therapieerfolg gefährdet wird. Dem Gerät liegt der therapeutische Gedanke zu Grunde, durch Wärmeanwendung Keime zu zerstören und Reizerreger der Haut abzutöten. Die hierfür benötigten Behandlungsdauern und/oder -temperaturen sind allerdings nicht geeignet, Juckreiz durch gezielte Stimulation bestimmter Rezeptoren und die Modifikation des Immunsystems nachhaltig zu lindern. Temperaturen unterhalb von 42 °C sind wiederum ungeeignet, über eine Wärmeempfindung hinaus Effekte therapeutischer Art zu erzielen.

US 2011/0184502 A1 beschreibt ein Heizkissen für diverse, teils medizinische Anwendungen, welches Temperaturen von 38 °C - 71 °C über mindestens mehrere Minuten elektrisch erzeugt. Es werden nicht rückstellende Thermosicherungen in Serie als redundantes Sicherheitsfeature vorgeschlagen. Somit ist zwar ein redundanter Sicherheitsmechanismus vorhanden, dieser ist jedoch nicht reversibel und muss nach dem Auslösen ausgetauscht werden. Ein zweiter Nachteil bei der Verwendung von thermischen Sicherungen ist, dass diese erst bei Anliegen einer Temperatur oberhalb eines Schwellenwerts abschmelzen. Somit reagieren thermische Sicherungen erst bei Anliegen dieser kritischen Temperatur nach einer gewissen Reaktionszeit und somit gegebenenfalls zu spät im Vergleich zu einer Schmelzsicherung. Eine Schmelzsicherung löst bereits bei einem elektrischen Strom oberhalb eines Schwellenwertes aus, der eine zu hohe Temperatur verursachen könnte, wenn er zu lange fließt. Darüber hinaus ist sowohl der Temperaturbereich als auch die Dauer des Heizprozesses für eine Vielzahl von Anwendungen sicherlich relevant, jedoch ungeeignet, Pruritus durch Hitzeanwendung nachhaltig zu lindern.

Zudem ist insbesondere im Stand der Technik kein Gerät bekannt, welches die Vorzüge moderner Mobilgeräte, beispielsweise hoher Rechenleistung, Vernetzung, aber auch die Fähigkeit, als Energieträger für physikalische Anwendung zu dienen, mit einer Vorrichtung zur Behandlung von den o.g. Juckreizerkrankungen auf sichere und zuverlässige Weise verbindet.

Mobilgeräte, insbesondere Smartphones, werden heute nicht nur für Ihre ursprüngliche Bestimmung, beispielsweise zum Telefonieren, verwendet. Es ist vielmehr heutzutage üblich, in den kleinen tragbaren Geräten so viele Anwendungen wie möglich zu vereinen. So werden heutige Smartphones ebenfalls als Fotoapparate, Abspielgeräte, Reader, zum Computerspielen, als Navigationsgerät und für vieles mehr verwendet.

Für medizinische Anwendungen, insbesondere bei der Behandlung von Juckreiz oder Herpeserkrankungen spielen Mobilgeräte bislang eine untergeordnete Rolle.

Es wäre somit wünschenswert eine Vorrichtung bereitstellen zu können, welche die Vorzüge der hyperthermischen Behandlung für die Vielzahl der genannten Erkrankungen umsetzt und gleichzeitig Sicherheitsrisiken minimiert.

### Aufgabe der Erfindung

Eine Aufgabe der Erfindung war es, die Nachteile des Standes der Technik zu beseitigen. Insbesondere war es Aufgabe der Erfindung eine Vorrichtung zur hyperthermischen Behandlung von Juckreiz oder Herpeserkrankungen bereitzustellen, welche die Vorzüge von Mobilgeräten, u.a. in Bezug auf deren Bedienerfreundlichkeit, Rechenleistung oder Vernetzbarkeit ausnutzen kann und gleichzeitig hohe Sicherheitsstandards gewährleistet.

### Zusammenfassung der Erfindung

Die Aufgabe der Erfindung wird durch eine Vorrichtung gemäß dem unabhängigen Anspruch gelöst. Die abhängigen Ansprüche betreffend bevorzugte Ausführungsformen der Erfindung.

In einer bevorzugten Ausführungsform betrifft die Erfindung somit eine Vorrichtung zur hyperthermischen Behandlung von Juckreiz und/oder Herpeserkrankungen umfassend
a. mindestens eine Behandlungsfläche
b. ein Bedienelement und
c. eine Regelvorrichtung, welche konfiguriert ist nach Betätigung des Bedienelementes durch Erhitzen mindestens eines Heizelements gemäß vorgegebener Behandlungsparameter eines Behandlungsprogrammes die Behandlungsfläche in einer Aufheizphase auf eine Behandlungstemperatur zwischen 40° - 65°C zu regulieren und für eine Behandlungsdauer zwischen 1 und 12 Sekunden zu halten
dadurch gekennzeichnet, dass
auf der Regelvorrichtung mindestens zwei unterschiedliche Behandlungsprogramme gespeichert vorliegen, welche unterschiedliche Behandlungsparameter festlegen und wobei die Vorrichtung mindestens ein Interface zur Verbindung mit einem Mobilgerät aufweist und die Vorrichtung konfiguriert ist für die Auswahl eines der mindestens zwei Behandlungsprogramme durch das Mobilgerät, sodass nach Betätigung des Bedienelement das ausgewählte Behandlungsprogramm ausgeführt wird.

Untersuchungen haben ergeben, dass das Einbringen von Wärme durch eine derartige Vorrichtung erfolgreich zur Behandlung von Juckreiz verwendet werden kann.

Dafür wird die erfindungsgemäße Vorrichtung bevorzugt auf die betroffenen Hautpartien aufgelegt. Nach der Kontaktierung der Hautpartie mit der Behandlungsfläche sorgt eine Regelvorrichtung dafür, dass eine erfindungsgemäße Regulation der Temperatur der Behandlungsfläche erfolgt. Die Behandlungstemperatur gibt dabei bevorzugt stets jene Temperatur an, welche an der Hautpartie des Patienten vorliegt. Bevorzugt wird die Behandlungsfläche hierzu zunächst in einer Aufheizphase auf eine Behandlungstemperatur zwischen 40 °C und 65 °C geführt.

Es ist bevorzugt, dass die Aufheizphase keinen längeren Zeitraum bedarf. Bevorzugt sollte die Aufheizphase nicht mehr als 12 sec, besonders bevorzugt nicht mehr als 3 sec betragen. In einer bevorzugten Ausführungsform der Erfindung beträgt die Aufheizphase 1 Sekunde bis 5 Sekunden, bevorzugt weniger als 3 Sekunden und insbesondere 1 bis 2 Sekunden. Durch so eine schnelle Aufheizphase kann besonders schnell die gewünschte Temperatur erreicht werden. Somit können bevorzugt Heilungseffekte erzielt werden, ohne einem Anwender unnötig Hitze zuzuführen und/oder die effektiv für eine Behandlung benötigte Zeit zu vergrößern. Außerdem kann so die abgegebene Wärmemenge während der Behandlung besonders präzise bestimmt werden.

Im Anschluss an die Aufheizphase wird die Temperatur der Behandlungsfläche bei der vorbestimmten Behandlungstemperatur für eine vorbestimmte Behandlungsdauer von 1 bis 12 Sekunden gehalten.

Durch die Regulation der Behandlungsfläche auf einer Temperatur zwischen 40°C und 65 °C während einer Behandlungsphase wird Wärme auf wohldefinierte Art übertragen. Hierdurch können Schmerzen und weitere unangenehme Empfindungen wie bspw. ein Ziehen oder Juckreiz überraschend wirksam gelindert werden. Der Effekt kann auch bei Insektenstichen oder Herpeserkrankungen zum Tragen kommen, bei denen die Linderung zusätzlich aus einer thermischen Neutralisation der Gifte der Insekten oder Herpes auslösenden Viren beruht.

Zum anderen bewirkt die Wärme eine Nervenstimulation, welche die subjektive Wahrnehmung von Schmerzen oder Juckreiz an den betreffenden Stellen stark reduziert. Die Wärmeübertragung führt überraschend zu einer Überlagerung der unangenehmen Empfindung durch andere temperaturabhängige Hautempfindungen.

Entgegen konventionellen Methoden zur Behandlung wird dabei zusätzlich eine Regulation der Schmerzrezeptoren anvisiert und durch die bevorzugte Wärmebehandlung die freien Nervenenden der C-Fasern aktiviert. Die C-Fasern bezeichnen insbesondere die langsam leitenden Nervenfasern des somatosensiblen Systems und sind für die Schmerzwahrnehmung verantwortlich. Hierbei kommt insbesondere den freien Enden der C-Fasern, welche auch als Nozizrezeptoren bezeichnet werden, eine wichtige Rolle zu. Die Nervenenden der Fasern werden durch Gewebshormone (z. B. Histamin, Serotonin, Substanz P) aktiviert. Auch könnten Mastzellen in der Nähe der Nervenenden durch Ausschüttung des Mediators Tryptase an dem Prozess beteiligt sein. Insbesondere wird die Erkenntnis über den Wirkmechanismus bei Juckreiz oder Herpes ausgenutzt, um durch eine Wärmebehandlung in überraschender Weise die Sinneswahrnehmung, welche durch die Fasern ausgelöst werden, zu regulieren.

Es wurde darüber hinaus erkannt, dass eine besonders starke Überlagerung der unangenehmen Empfindungen erreicht werden kann, wenn in den betreffenden Hautpartien lokal die Thermo- und Capsaicinrezeptoren TRPV1 und TRPV2 zeitgleich aktiviert werden. Der TRPV1 ist bei akutem Hitze-induzierten Schmerz in gesunder Haut beteiligt und reguliert beispielsweise das Heißempfinden bei Temperaturen um 45° bis 50 °C. Bei besonders starken schmerzhaften Hitzereizen, welche ab Temperaturen von über 52 °C eintreten wird darüber hinaus der TRPV2 aktiviert. Die Aktivierungsschwelle des TRPV1 liegt zwischen 40°C und 45°C, wohingegen die des TRPV2 zwischen 50°C und 53° beträgt (Yao et al 2011, Somogyi et al. 2015, Cohen et al. 2014, Mergler et al. 2014).

Im Sinne der Erfindung bezeichnet der Begriff "Behandlungsparameter" bevorzugt jene Parameter oder Kenngrößen, welche den Verlauf der Temperaturregulation der Behandlungsfläche während einer Behandlung kennzeichnen. Bevorzugte Behandlungsparametern sind bevorzugt die Behandlungstemperatur, die Behandlungsdauer oder auch die Dauer einer Aufheizphase. Aber auch andere Parameter können Behandlungsparameter darstellen. Falls bei der Vorrichtung eine separate Vorheizphase vorgesehen ist, um die Behandlungsfläche zunächst auf eine Vorheiztemperatur unterhalb der Behandlungstemperatur zu bringen, kann die Dauer der Vorheizphase oder Vorheiztemperatur weitere Behandlungsparameter darstellen. Bevorzugt betreffen die Behandlungsparameter somit jegliche Parameter, welche die Temperatur der Behandlungsfläche zu einem Zeitpunkt beeinflussen können.

Ein "Behandlungsprogramm" bezeichnet bevorzugt einen Algorithmus, welcher auf Basis definierter Behandlungsparameter die Regulation des Temperaturverlaufes der Behandlungsfläche durch die Regelvorrichtung vorgibt. Das Behandlungsprogramm umfasst somit bevorzugt eine definierte Kombination von Behandlungsparameter sowie ggf. zusätzlichen Regelalgorithmen zur Gewährleistung der vorgebenden Behandlungsparameter. Beispielsweise kann es bevorzugt sein, die Behandlungstemperatur über einen Temperatursensor in einem vorgebenden Bereich feedbackgesteuert zu halten. In einem solchen Fall kann das Behandlungsprogramm bevorzugt neben einer vergebenen Behandlungstemperatur für die Behandlungsfläche zusätzliche Anweisung zur Feedbackregulation der Behandlungstemperatur umfassen.

In einer bevorzugten Ausführungsform umfassen die durch die Behandlungsprogramme festgelegten Behandlungsparameter die Behandlungstemperatur und die Behandlungsdauer, wobei bevorzugt zusätzlich eine Dauer für die Aufheizphase als Behandlungsparameter festgelegt wird. Diese Parameter sind besonders behandlungsrelevant und definieren den Temperaturverlauf, welcher für den Behandlungserfolg wesentlich ist.

Das Behandlungsprogramm kann dabei softwarebasiert und/oder hardwarebasiert sein. Die Vorrichtung zeichnet, sich dadurch aus, dass auf Regelvorrichtung mindestens zwei Behandlungsprogramme gespeichert vorliegen. Besonders bevorzugt unterscheiden sich die mindestens zwei Behandlungsprogramme in mindestens einem Behandlungsparameter. Beispielsweise kann ein erstes Behandlungsprogramm eine erste Behandlungstemperatur vorgeben, während ein zweites Behandlungsprogramm eine zweite Behandlungstemperatur vorgibt. Gleichermaßen können sich die Behandlungsprogramme selbstverständlich auch in der Behandlungsdauer, der Dauer der Aufheizdauer oder einem anderen Behandlungsparameter unterscheiden. Auch können sich die Behandlungsprogramme in zwei oder der Behandlungsparameter unterscheiden.

Durch die Möglichkeit der Auswahl verschiedener Behandlungsprogramme kann beispielsweise die Vorrichtung zu unterschiedlichen Behandlungszwecken optimal verwandt werden. So kann ein erstes Behandlungsprogramm für die Behandlung von Lippenherpes optimiert sein, während ein zweites Behandlungsprogramm besonders effektive zur Verminderung von Juckreiz nach Insektenstichen führt.

Auch können die unterschiedlichen Behandlungsprogramme dafür genutzt werden, um die Behandlung an die Bedürfnisse und Präferenzen unterschiedlicher Anwender anzupassen.

Untersuchungen haben gezeigt, dass die individuelle Empfindung der Hitzebehandlung von Anwender zu Anwender unterschiedlich wahrgenommen wird. Einige Probanden empfinden beispielsweise eine Temperatur von oberhalb von 48°C bereits als schmerzhaft, während andere Probanden auch Temperatur von 50°C oder mehr als angenehm und Juckreiz mindert beschrieben. Durch die Bereitstellung von mindestens zwei Behandlungsprogrammen kann daher eine individuellere Behandlung erfolgen und der Behandlungserfolg erhöht werden.

Zur Auswahl des Behandlungsprogrammes umfasst die Vorrichtung mindestens ein Interface, welches für die Auswahl eines der mindestens zwei Behandlungsprogramme durch ein Mobilgerät konfiguriert ist.

Unter einem Mobilgerät werden bevorzugt mobile Endgeräte verstanden, die aufgrund ihrer Größe und ihres Gewichts ohne größere körperliche Anstrengung tragbar und somit mobil einsetzbar sind. Bevorzugt handelt es sich um elektronische Endgeräte für mobile, netzunabhängige Daten-, Sprach- und Bildkommunikation, Navigation oder dergleichen. Besonders bevorzugte Mobilgeräte sind beispielsweise ein Laptop, ein Mobiltelefon, ein Smartphone, ein Tablet-Computer, ein Notebook und/oder eine Smartwatch sein.

Derartige Mobilgeräte erfreuen sich hoher Beliebtheit und zeichnen durch besonders ansprechende Bedienmöglichkeiten und hohe Bedienerfreundlichkeit aus. Eine Einbindung der Mobilgeräte zur Bedienung einer hyperthermischen Behandlungsvorrichtung führt zu einer Reihe von Vorteilen. Zum einen kann eine Auswahl bevorzugten Behandlungsprogramme übersichtlicher und leichter erfolgen, wobei das Mobilgerät dem Nutzer auch bisherige erfolgreiche Behandlungsprogramme anzeigen kann. Auch können auf dem Mobilgerät mehrere Nutzerprofile hinterlegt werden, welche jeweils die bevorzugten Behandlungsprogramme für den jeweiligen Nutzer aufzeigen. Es zeigt sich, dass durch Einbindung eines Mobilgerätes die Behandlungshäufigkeit und somit auch der Behandlungserfolg auf deutliche Weise steigern lässt.

Zudem lässt sich durch die Möglichkeit der Verbindung mit einem Mobilgerät eine besonders kompakte Vorrichtung zur Behandlung von Juckreiz und/oder Herpeserkrankungen realisieren. Beispielsweise ist nicht notwendig, dass die Vorrichtung selbst über viele Bedien- oder Anzeigenelemente verfügt. Stattdessen können Funktionen wie die Auswahl von Behandlungsprogrammen, die Anzeige einer erfolgreichen Behandlung, der Temperaturverlauf der Behandlungsfläche oder andere Funktionen, welche die Durchführung und Kontrolle der Behandlung erleichtern von dem Mobilgerät übernommen werden. Auch muss die Vorrichtung selbst nicht über hohe Rechenleistung oder Regelkapazitäten verfügen. Stattdessen kann die Regelvorrichtung im Wesentlichen dafür ausgelegt werden sicherheitsrelevante Funktionen wie die Regulation der Temperatur der Behandlungsfläche vorzunehmen. Eine längerfristige Speicherung von Daten oder Berechnungen auf Basis solcher Daten können vorteilhafterweise auch an ein Mobilgerät ausgelagert werden.

Es lässt sich somit eine besonders kompakte, kostengünstige und effiziente Vorrichtung zur Behandlung von Juckreiz oder Herpeserkrankungen bereitstellen, welche gleichzeitig von den umfangreichen Bedien-, Anzeigen- und Rechenkapazitäten moderner Mobilgeräte profitiert.

Erfindungsgemäß kann die Verbindung der Vorrichtung über das vorhandene Interface erfolgen.

Das Vorhandensein eines Interface oder, synonym, eine Schnittstelle, bedeutet bevorzugt, dass die Vorrichtung dafür konfiguriert ist, mit einem Mobilgerät zu interagieren und/oder verbunden zu werden.

Bei dem Interface kann sich um einen physischen Ort handeln, an dem eine Verbindung der Vorrichtung mit einem anderen Gerät möglich ist. Es kann sich aber ebenso um andere, optionale Verbindungsmöglichkeit der Vorrichtung handeln, mit einer anderen Vorrichtung Daten, Signale und/oder Energie auszutauschen. Dabei kann es sich bevorzugt um elektrische Signale handeln oder um Signale, welche von der Vorrichtung in elektrische Signale umgewandelt werden können.

Eine Verbindung, welche über eine solche Schnittstelle oder Interface ermöglicht wird, umfasst bevorzugt drei Elemente: eine Schnittstelle des Mobilgeräts, die Schnittstelle der Vorrichtung sowie einen Übertragungskanal zwischen diesen beiden. Der Übertragungskanal kann beispielsweise eine kabelbasierte Verbindung sein und/oder ein drahtloser Übertragungskanal. Es kann auch bevorzugt sein, dass zwei Schnittstellen in Form von Buchse und dazu passendem Stecker ausgeführt sind und so eine direkte Verbindung herstellbar ist, welche bevorzugt des Weiteren eine mechanisch stabile Verbindung zwischen Vorrichtung und Mobilgerät ermöglicht. In diesem Fall und bei einer kabelbasierten Verbindungsmöglichkeit würde die Schnittstellte der Vorrichtung bevorzugt eine Buchse und/oder einen Stecker umfassen.

Je nachdem, ob die Schnittstelle drahtlos oder kabelgebunden ist oder für eine direkte Steckverbindung mit Stecker und Buchse vorgesehen ist, umfasst die Schnittstelle, unterschiedlich gestaltete Elemente.

Bei der Schnittstelle kann es sich um eine standardisierte Schnittstelle handeln, wie beispielsweise Bluetooth, Lightning, USB, WLAN etc. Es kann sich jedoch auch um eine individuell für die Vorrichtung entwickelte Schnittstelle handeln.

Die Schnittstelle kann drahtlos oder kabelgebunden sein. Ein Kabel kann sowohl zur Übertragung elektrischer Ladungsträger geeignet sein, beispielsweise ein Kupferkabel sein, es kann aber ebenso ein Kabel beispielsweise zur Übertragung optischer Signale, zum Beispiel ein Glasfaserkabel verwendet werden. Eine drahtlose Übertragung kann bevorzugt auf der Übertragung elektromagnetischer Wellen im kompletten Spektralbereich basieren, es können beispielsweise Lichtsignale zur drahtlosen Übertragung verwendet werden, aber ebenso Kurzwellen, Ultrakurzwellen und/oder Dezimeterwellen.

Handelt es sich bei der Schnittstelle z.B. um USB, ist mit Schnittstelle der Vorrichtung bevorzugt eine zur Verbindung geeignete USB-Buchse der Vorrichtung gemeint. Des Weiteren ist bevorzugt gemeint, dass von Seiten der Vorrichtung geeignete Datenwandler, Leitungstreiber, Stromversorgungen und/oder Prozessoren vorhanden sind, um die USB-Buchse bestimmungsgemäß zu nutzen, insbesondere zur Einrichtung einer USB-Verbindung mit einem Mobilgerät. Außerdem soll insbesondere eine Verbindung von elektrischen Komponenten der Vorrichtung mit der Schnittstelle gewährleistet sein. Dieses Beispiel dient der Illustration einer Schnittstelle der Vorrichtung und ist bevorzugt vom Prinzip her auf andere Arten von Schnittstellen der Vorrichtung übertragbar.

Die Schnittstelle der Vorrichtung ist bevorzugt mindestens für die Aufnahme von Daten oder Informationen konfiguriert, welche eine Auswahl eines der mindestens zwei Behandlungsprogramme erlauben.

Zum Empfangen von Daten kann die Vorrichtung eine Datenempfangseinheit umfassen, welche auch in der Schnittstelle integriert vorliegen kann. Die Datenempfangseinheit kann vom Mobilgerät kommende Daten über die Schnittstelle empfangen, diese gegebenenfalls in ein geeignetes Datenformat umwandeln und an die Regelvorrichtung zur Weiterverarbeitung und/oder ein Speichermedium zum Speichern in geeigneter Weise weitergeben. Ein Fachmann kennt Möglichkeiten, geeignete Einheiten routinemäßig zu realisieren.

Die Vorrichtung, bzw. deren Regelvorrichtung und Schnittstelle, ist bevorzugt, derart konfiguriert dass eine Auswahl eines der mindestens zwei Behandlungsprogramme durch ein Mobilgerät erfolgen kann. Die entsprechende Information der Auswahl wird hierbei bevorzugt vom Mobilgerät über die Schnittstelle an die Regelvorrichtung übertragen.

Die Auswahl meint dabei bevorzugt, dass die Behandlungsprogramme auf der Regelvorrichtung gespeichert vorliegen und die Datenübertragung durch das Mobilgerät festlegen kann, welches der gespeicherten Behandlungsprogramme bei Betätigung des Bedienelementes ausgeführt wird.

Bei einer derartigen Auswahl ist es insbesondere nicht möglich, dass das Mobilgerät über die Schnittstelle eine Veränderung der gespeicherten Behandlungsprogramme vornimmt. D.h. sowohl eine Änderungen der Behandlungsparameter, als auch die Zusammenstellung eines neuen Behandlungsprogrammes als Kombination hinterlegter Behandlungsparameter durch ein Mobilgerät ist nicht vorgesehen. Durch entsprechende Schaltung werden derartige Änderungen bevorzugt ausgeschlossen.

In Bezug auf die Regulation der Temperatur ist die Vorrichtung somit autark und unabhängig von dem Mobilgerät.

Erfindungsgemäß wurde erkannt, dass durch eine derartige Beschränkung der Regulationsmöglichkeiten eines Mobilgeräts sich besonders hohe Sicherheitsstandards gewährleisten lassen.

Ein alternativer Ansatz bestünde darin durch das Mobilgerät behandlungsrelevanter Parameter über die Schnittstelle auf die Regelvorrichtung der Vorrichtung zu übertragen. Vordergründig ließen sich dadurch individuelle Behandlungsprogramme zusammenstellen, um das Behandlungserlebnis zu verbessern. Die Erfinder haben jedoch erkannt, dass eine sichere Durchführung der hyperthermischen Behandlung hierdurch stark beeinträchtigt werden kann.

Mobile Endgeräte zeichnen sich durch einen hohen Grad an Vernetzbarkeit aus und verfügen typischerweise über eine Vielzahl verschiedener Software (,Apps') mit unterschiedlicher Herkunft und Funktion. Beide Umstände bedingen, dass mobile Endgeräte als offene Systeme inhärent anfällig für Sicherheitsrisiken sind.

So kann es vorkommen, dass nach einem Update der Betriebssoftware eine Software zur Verbindung mit der Vorrichtung nur noch fehlerhaft ausgeführt wird. Auch eine gezielte Manipulation durch Computerviren oder andere Hackerattacken kann nicht ausgeschlossen werden.

Die Bereitstellung einer externen Software auf einem Mobilgerät, welches derartige Szenarien ausschließen kann, wäre mit einem hohem Aufwand und Kosten verbunden. Ein Restrisiko bestünde zudem weiterhin.

In Abkehr eines derartigen Ansatzes wird durch die erfindungsgemäß vorgesehene Beschränkung der Regulationsrechte des Mobilgerätes auf eine Auswahl vorgegebener Behandlungsprogramme ein besonders hoher Sicherheitsstandard auf einfache und kosteneffiziente Weise umgesetzt.

Da eine Software auf dem Mobilgerät keinen Einfluss auf die sicherheitsrelevanten Behandlungsparameter der Vorrichtung nehmen kann, kann die Software deutlich niedrigeren Sicherheitsstandards genügen, ohne dass sich dies auf die Sicherheit des Vorrichtung zur hyperthermische Behandlung von Juckreiz oder Herpeserkrankungen auswirken können.

Selbst bei einem Ausfall der Bediensoftware auf dem Mobilgerät oder einem gezielten Manipulationsversuch kann über die Schnittstelle der Vorrichtung stets nur eine Auswahl der vorinstallierten Behandlungsprogramme erfolgen.

In einer bevorzugten Ausführungsform ist die Vorrichtung somit derart konfiguriert, dass eine Änderung der Behandlungsparameter, welche durch die Behandlungsprogramme festgelegt werden, durch das Mobilgerät ausgeschlossen ist. Dem Fachmann sind zu diesem Zweck verschiedene technische Lösungen bekannt.

Beispielsweise können die Behandlungsprogramme in der Firmware der Regelvorrichtung derart vorgegeben sein, sodass eine Änderung über die Schnittstelle grundsätzlich ausgeschlossen wird. Im Sinne der Erfindung wird bevorzugt unter der Firmware eine Software, d.h. die Anleitung für ein computer-implementiertes Verfahren verstanden, welches in der Regelvorrichtung bevorzugt in einem Mikroprozessor eingebettet vorliegt. D.h. die Firmware umfasst bevorzugt jene Software, welche mit der Hardware der Vorrichtung, d.h. insbesondere mit dem mindestens einen Heizelement und ggf. Temperatursensoren funktional verbunden ist. Vorzugsweise wird die Firmware mit dem Start der Vorrichtung ausgeführt und übernimmt die Kontroll- und Steuerungsfunktion dieser Hardware-Komponenten der Vorrichtung.

In der Firmware kann somit bevorzugt lediglich ein Parameter für die Auswahl eines Behandlungsprogrammes durch eine Ansteuerung über die Schnittstelle änderbar sein, nicht aber das hinterlegte Behandlungsprogramm als solches.

Die Behandlung selbst wird somit unabhängig von einer Verbindung mit einem Mobilgerät stets in den von den Behandlungsprogrammen durch die Behandlungsparameter festgelegten Bereichen erfolgen.

Die Behandlungstemperatur entspricht dabei bevorzugt einer konstanten Temperatur, welche im genannten Bereich zwischen 40 °C und 65 °C liegt. Diese Behandlungstemperatur wird während der Behandlungsphase bevorzugt konstant gehalten. Konstant umfasst hierbei bevorzugt auch eine Schwankung der Temperatur innerhalb eines vorgegebener Toleranzbereich, beispielsweise von ± 10%, ± 5%, ± 3% oder ± 1% oder weniger.

Die Behandlungsphase bezeichnet bevorzugt den Zeitraum, bei welchem die Temperatur der Behandlungsfläche im Bereich der Behandlungstemperatur von 40 °C bis 65 °C ist. Bevorzugt dauert die Behandlungsphase 1 bis 12 sec, besonders bevorzugt dauert die Behandlungsphase zwischen 2 sec und 12 sec, 1 sec und 10 sec, ganz besonders bevorzugt zwischen 3 sec und 5 sec. Es ist besonders bevorzugt, dass die Behandlungsphase einen zusammenhängenden Zeitraum bezeichnet. Es kann aber auch sein, dass die Behandlungsphase durch das Führen der Temperatur, welches zeitlich die Form einer Rampe aufweist, kurzzeitig unterbrochen wird. In diesem Falle wird als Zeitraum der Behandlungsphase bevorzugt diejenige Zeit verstanden, während welcher die Temperatur der Behandlungsfläche im Bereich der Behandlungstemperatur liegt. Nach Beendigung der Behandlungsphase erfolgt bevorzugt kein Erhitzen des mindestens eines Heizelementes durch die Regelungsvorrichtung mehr, sodass die Temperatur der Behandlungsfläche unter die Behandlungstemperatur fällt.

Im Sinne der Erfindung bezeichnet die Behandlungsfläche bevorzugt diejenige Fläche der Vorrichtung, welche während der Behandlung auf die Behandlungstemperatur aufgeheizt wird und in direktem thermischen Kontakt mit der Hautpartie steht. Die Behandlungsfläche kann eine zusammenhängende Fläche darstellen. Es kann auch bevorzugt sein, dass die Behandlungsfläche aus mehreren nicht zusammenhängenden Teilflächen besteht. Die Größe der Behandlungsfläche hängt bevorzugt von der Erkrankung und der Größe der von den Symptomen der Juckreizerkrankung betroffenen Hautpartien ab. Bei Insektenstichen ist die Größe der Behandlungsfläche bevorzugt zwischen 10 mm² und 100 mm², besonders bevorzugt zwischen 20 mm² und 60 mm². Bei der Behandlung von Herpes beträgt die Behandlungsfläche bevorzugt zwischen 10 mm² und 80 mm², besonders bevorzugt zwischen 20 mm² und 50 mm². Weiterhin ist es besonders bevorzugt, dass die Behandlungsfläche für diese kleineren Hautpartien kreisförmig ist. Durch die so gewählten Größen und Geometrien der Behandlungsfläche kann eine der Ursache optimal angepasste Behandlung erfolgen, welche Effizienz und Wohlbefinden optimiert und so zu einem nachhaltigeren Behandlungserfolg beiträgt.

Die Größe der Behandlungsfläche bezieht sich bevorzugt jeweils auf die gesamte Kontaktfläche, über welche eine Hautpartie einen Wärmeimpuls erfährt. Im Falle einer Behandlungsfläche, welche aus mehreren Teilflächen besteht, entspricht die Größe der Behandlungsfläche bevorzugt der Summe der einzelnen Teilflächen. Eine solche Aufteilung in Teilflächen kann bei bestimmten Erscheinungsformen von Herpes oder Juckreiz vorteilhaft sein, ebenso wie bei der Behandlung bestimmter Körperstellen.

Es ist bevorzugt, dass die Behandlungsfläche mit Hilfe von mindestens einem Heizelement auf die gewünschte Behandlungstemperatur gebracht wird. In einer bevorzugten Ausführungsform entspricht die Behandlungsfläche der Oberfläche einer Heizplatte, welche mit Hilfe eines Heizelementes erhitzt wird, wobei zum Beispiel ein Halbleiter-Bauelement zum Einsatz kommen kann. Die Behandlungsfläche kann auch eine homogene Materialfläche bezeichnen, welche durch mehrere Heizelemente temperiert wird. Beispielsweise kann es bevorzugt sein, zwei oder vier Heizelemente zu verwenden, um die Behandlungsfläche besonders homogen und schnell auf die Behandlungstemperatur zu führen.

Es ist bevorzugt, dass eine Regelvorrichtung das Erhitzen des Heizelementes derart regulieren kann, dass die Behandlungstemperatur an der Behandlungsfläche vorliegt. So kann eine optimale Kontrolle der Behandlungstemperatur gewährleistet werden und ein ungewünschtes Überhitzen der Behandlungsfläche unterbunden werden.

Im Sinne der Erfindung ist die Regelvorrichtung bevorzugt ein integrierter Schaltkreis, Prozessor, ein Prozessorchip, Mikroprozessor, Mikrochip oder ein Mikrokontroller, welcher konfiguriert ist, um die Temperatur der Behandlungsfläche mit Hilfe des mindestens einen Heizelements gemäß der vorgegebenen Behandlungsparameter zu regulieren. Eine solche Regelvorrichtung zeichnet sich durch Kompaktheit, Zuverlässigkeit, Kosteneffizienz, geringen Stromverbrauch und hohe Regeleffizienz aus. Erfindungsgemäß ist die Regelvorrichtung von der Vorrichtung umfasst. Bevorzugt wird unter dem Heizelement jenes Bauelement bezeichnet, welches durch die Regelvorrichtung u.a. durch Anlegen eines elektrischen Stromes erhitzt werden kann. Das mindestens eine Heizelement ist bevorzugt ein Bauelement, für welches verschiedene Ausführungsformen aus dem Stand der Technik hinreichend bekannt sind. So kann das Heizelement einen Leistungswiderstand umfassen, bei welchem in Abhängigkeit des Stromflusses eine wohldefinierte Temperatur generiert wird. Bevorzugt kann zur quantitativen Steuerung des Stromflusses durch das Heizelement ein Feldeffekttransistor (FET) verwandt werden. Es kann aber auch bevorzugt sein, einen FET selbst als Heizelement einzusetzen. Hierbei wird die Energiedissipation im Transistor genutzt, um Wärme zu generieren und die Behandlungsfläche auf die Behandlungstemperatur zu bringen. FETs sind als Heizelemente besonders bevorzugt, da diese aufgrund ihrer geringen Größe eine kleine Dimensionierung erlauben. Weiterhin sind FETs besonders reaktiv und gewährleisten durch eine sehr dynamische Wärmeerzeugung und Wärmeabgabe ein besonders schnelles Ansprechverhalten der Heizelemente.

Bevorzugt kann die Regelvorrichtung durch die Vorgabe der Stromzufuhr zum Heizelement kontrollieren, welche Temperatur an der Behandlungsfläche vorliegt. Beispielsweise kann mit Hilfe einer Kalibration die Korrelation zwischen Stromfluss und/oder Spannung an dem Heizelement mit der Temperatur an der Behandlungsfläche ermittelt werden, sodass auf Basis der Kalibrierung stets eine gewünschte Behandlungstemperatur zwischen 40 °C und 65 °C eingestellt werden kann. Es kann aber auch bevorzugt sein, die Behandlungstemperatur durch die Regelvorrichtung mit Hilfe einer Feedbackschleife zu regulieren. So kann es bevorzugt sein, einen Temperatursensor einzusetzen, welcher die Temperatur der Behandlungsfläche misst, wobei die Regelvorrichtung anhand der Temperaturdaten die Stromzufuhr zum Heizelement reguliert. Zu diesem Zweck kann die Regelvorrichtung beispielsweise einen Mikroprozessor umfassen, welcher Messdaten auswerten und Stromparameter festlegen kann. So kann die Temperatur sehr effizient und zuverlässig gesteuert werden.

Im Sinne der Erfindung wird unter einem Mikroprozessor bevorzugt eine Datenverarbeitungsvorrichtung, d.h. ein Prozessor, verstanden, welcher sich durch kleine Abmaße im Bereich von einigen mm auszeichnet und wobei bevorzugt alle Bausteine des Prozessors auf einem Mikrochip oder auch integriertem Schaltkreis (engl. *integrated circuit, IC*). Der Mikroprozessor kann bevorzugt auch ein Mikrokontroller sein, welcher neben dem Prozessor weitere periphere Elemente auf dem Mikrochip integriert und beispielsweise auch über einen Datenspeicher verfügt.

Es ist weiterhin bevorzugt, dass der Mikroprozessor auf einer Leiterplatte (engl. *printed circuit board,* PCB) installiert vorliegt. Außer dem Mikroprozessor liegen auf dem PCB weiterhin bevorzugt die Heizelemente sowie die Temperatorsensoren installiert vor. Diese bevorzugte Ausführungsform erlaubt eine äußert kompakte und ebenso robuste Bauweise der Vorrichtung und eine besonders intelligente Temperaturregulation mit Hilfe des Mikroprozessors. So ist der Mikroprozessor nicht nur in der Lage die gemessenen Temperaturdaten auszuwerten und in einer Steuerung der Heizelemente zu übersetzen, sondern kann weiterhin andere Parameter wie Fehlermeldungen und Benutzerinput schnell und zuverlässig berücksichtigen.

In einer bevorzugten Ausführungsform der Erfindung ist die Vorrichtung dadurch gekennzeichnet, dass der Mikroprozessor und das Heizelement und optional ein Temperatursensor auf einem *printed circuit board* (PCB) installiert sind, wobei mindestens das Heizelement und der Temperatursensor mit Hilfe eines Schutzlackes beschichtet sind. Im Sinne der Erfindung wird unter dem Schutzlack bevorzugt ein Lack oder eine Farbe verstanden, welcher Bauteile des PCBs vor Umwelteinflüssen schützen soll.

Der Schutzlack wirkt zu diesem Zweck bevorzugt elektrisch isolierend und ist wasserbeständig. Die Eigenschaft der elektrischen Isolation kann bevorzugt anhand des Oberflächenwiderstandes oder engl. *surface insulation resistance* (SIR) quantifiziert werden. Der SIR kann bevorzugt beispielsweise durch Leckströme zwischen den Bauteilen der Leiterplatte gemessen werden. Ein hoher Widerstand entspricht einer guten elektrischen Isolation. Wasserbeständig meint bevorzugt, dass auch bei einer hohen Luftfeuchtigkeit oder dem Eindringen von Wasser die lackierten elektronischen Bauteile intakt bleiben und es zu keinem Kurzschluss kommt. Die Wasserbeständigkeit kann beispielsweise ebenfalls unter Messung der SIR unter Bedingungen mit hoher Luftfeuchtigkeit getestet werden.

Im Stand der Technik ist eine Vielzahl von Schutzlacken bekannt, welche bevorzugt verwandt werden können. Beispielhaft seien Schutzlacke auf Acryl-, Silikon- oder Polyurethanbasis genannt. Durch die Applikation des Schutzlackes im Bereich der Heizelemente und Temperatursensoren werden diese vor Ablagerungen wirksam geschützt, sodass Fehlmessungen der Temperatursensoren vermieden werden können. Dies erhöht zum einen die Genauigkeit mit welcher die Behandlungstemperatur eingestellt werden kann und vermeidet zum anderen, dass aufgrund einer Fehlmessung der Temperatur es zu einem Überhitzen der Behandlungsfläche kommt.

In einer bevorzugten Ausführungsform sind auf der Regelvorrichtung zwischen 2 und 20, 2 und 10 bevorzugt zwischen 4 und 8 Behandlungsprogramme gespeichert. Die Anzahl der Behandlungsprogramme hat sich als besonders vorteilhaft erwiesen. Einerseits können hierdurch hinreichend viele verschiedene für den Benutzer oder die Anwendung angepasste Behandlungen bereitgestellt werden. Anderseits ist die Anzahl ausreichend klein, um eine übersichtliche Auswahl mittels des Mobilgerätes zur erlauben und somit die Bedienerfreundlichkeit und Compliance zu erhöhen.

In einer weiteren Ausführungsform weist die Vorrichtung nur genau ein Bedienelement für das Erhitzen der Behandlungsfläche gemäß des ausgewählten Behandlungsprogrammes auf und an der Vorrichtung selbst kann manuell keine Auswahl des Behandlungsprogrammes erfolgen. Auch diese Ausführungsform erfreut sich aufgrund der schlichten Ausgestaltung einer besonders hohen Beliebtheit bei den Anwendern. Fehleinstellungen aufgrund verschiedener Bedienelemente, welche an der Vorrichtung selbst nur ungenügend erklärt werden können, werden wirksam vermieden.

Zudem zeichnet sich die Ausführungsform durch eine besonders kompakte und robuste Bauweise aus. Insbesondere beim Transport der Vorrichtung beispielsweise steigt das Risiko von Fehleinstellungen oder Abnutzungserscheinungen mit der Anzahl der Bedienelemente. Durch die Verwendung lediglich eines Bedienelementes, welches bevorzugt besonders robust gegen mechanische Einflüsse ausgestaltet ist, können diese Risiken reduziert werden.

Unter einem Bedienelement wird im Sinne der Erfindung bevorzugt jegliches Element verstanden, welches es erlaubt mittels einer manuellen Betätigung ein elektrisches Signal zu generieren bzw. einen Stromkreis zu schließen. Beispielsweise kann es sich um einen Tastschalter oder Bedienknopf handeln, welcher durch ein Drücken betätigt einen elektrischen Schaltkreis schließt und nach dem Loslassen selbsttätig in die Ausgangslage zurückkehrt. Auch andere elektrische Schalter wie Wippschalter, Rastschalter, Drehschalter etc. können bevorzugt verwandt werden. Derartige Schalter sind besonders robust und stellen einfache Bedienelemente dar, welche Fehleinstellungen verhindern. Das Bedienelement kann ebenfalls durch einen kapazitiven Sensor gebildet werden. Beispielsweise kann es sich um ein Touchpad, eine kapazitive Folientastatur oder andere kapazitive Sensoren handeln. Bekanntermaßen erkennen diese Berührungssensoren das Auflegen eines Fingers oder einer Hand auf Basis der Veränderung der Kapazität eines einzelnen Kondensators oder Kondensatorsysteme. Vorteilhafterweise können die Sensoren flächig auf der Vorrichtung aufgebracht werden, wodurch eine einfache manuelle Bedienung zur Auslösung der Behandlung erfolgen kann.

Die Vorrichtung ist bevorzugt derart konfiguriert, dass das nach manueller Betätigung des Bedienelementes ein Behandlungszyklus ausgelöst wird, d.h. die Regelvorrichtung gemäß eines ausgewählten Behandlungsprogrammes die Temperatur der Behandlungsfläche reguliert.

Vorteilhafterweise bedarf es hierzu keiner Verbindung mit einem Mobilgerät. Stattdessen kann die Vorrichtung in Bezug auf eigentliche Durchführung der Behandlung vollständig autark agieren.

In einer bevorzugten Ausführungsform ist die Vorrichtung derart konfiguriert ist, dass unabhängig von einer bestehenden Kommunikation zu einem Mobilgerät bei Betätigung ein zuletzt ausgewähltes Behandlungsprogramm ausgeführt wird.

Demgemäß ist die Regelvorrichtung bevorzugt derart konfiguriert, dass stets ein Behandlungsprogramm als aktuell ausgewählt gilt. Über die Schnittstelle kann bei bestehender Verbindung zu einem Mobilgerät selbiges ein neues Behandlungsprogramm auswählen. Sobald die Auswahl erfolgt ist, wird jenes Behandlungsprogramm als ausgewählt definiert. Selbst wenn im Anschluss die Verbindung abbricht, kann die Vorrichtung weiterhin mittels dieses zuletzt ausgewählten Behandlungsprogrammes eine Behandlung durchführen.

Die Vorrichtung ist somit vorteilhafterweise autark und bedarf nicht des Anschlusses oder der Nähe eines Mobilgerätes. Hierdurch kann ein besonders flexibler Einsatz der Vorrichtung auf Reisen oder im Freizeitbereich erfolgen, ohne dass stets ein Mobilgerät mitgeführt werden muss. Die Behandlung kann somit stets schnell und an Ort und Stelle durchgeführt werden, ohne dass eine Konfiguration oder Bestätigung mittels eines Mobilgerätes notwendig wäre.

In einer bevorzugten Ausführungsform ist das Interface und die Regelvorrichtung so konfiguriert sind, dass nur bei erfolgreicher Authentifizierung eine Auswahl eines der mindestens zwei Behandlungsprogramme mittels eines Mobilgerätes ermöglicht wird. Das Mobilgerät kann bevorzugt erkannt und authentifiziert werden, was insbesondere durch die Vergabe einer eindeutigen Kennung ermöglicht wird. Beispielsweise kann es erforderlich sein, dass das Mobilgerät neben Daten über die Auswahl eines Behandlungsprogrammes auch eine Kennung übermitteln muss, damit die Vorrichtung die entsprechende Auswahl akzeptiert. Dabei kann die Authentifizierung auch bevorzugt durch dem Fachmann bekannte kryptografische Mittel vorgenommen werden, wie sie bspw. bei Public-Key Infrastrukturen verwendet werden. Auch können digitale Zertifikate und/oder digitale Unterschriften zum Einsatz kommen. Eine derartige Authentifizierung kann zusätzlich unerlaubte externe Zugriffe auf die Vorrichtung verhindern. Beispielsweise könnte so auch sichergestellt werden, dass nach Diebstahl der Vorrichtung der Dieb nicht mit einem beliebigen Mobilgerät die Vorrichtung ansteuern kann.

In einer bevorzugten Ausführungsform ist die Vorrichtung dadurch gekennzeichnet, dass die Auswahl der Behandlungsprogramme durch das Mobilgerät über das Interface drahtlos erfolgt.

Eine drahtlose Übertragung von Signalen über die Schnittstelle kann beispielsweise in Form elektromagnetischer Wellen geschehen. Dafür würde die Schnittstelle bevorzugt mindestens eine Empfangseinheit und/oder eine Sendeeinheit umfassen. Ein Fachmann weiß, wie er die Schnittstelle mit geeigneten Sendern und/oder Empfangsantennen, Treibern und Datenwandlern ausstatten müssten sowie welche Verschaltungen zwischen Schnittstelle und weiteren elektrischen Elementen der Vorrichtung notwendig sind, damit eine drahtlose Übertragung gewährleistet werden kann. Beispielsweise kann eine drahtlose Übertragung über die Bluetooth-Technologie erfolgen, wobei die Schnittstelle über ein entsprechendes Bluetooth-Modul verfügt. Auch kann eine drahtlose Übertragung über Wi-Fi vorgesehen sein, wobei auf der Vorrichtung eine entsprechende WLAN Funkschnittstelle vorliegt.

Mittels der drahtlosen Übertragung kann auf besonders bequeme Weise durch das Mobilgerät eine Auswahl des Behandlungsprogrammes erfolgen.

In einer bevorzugten Ausführungsform betragen die in den mindesten zwei Behandlungsprogrammen festgelegten Behandlungstemperaturen zwischen 40 °C bis 60 °C, bevorzugt zwischen 45°C und 53°C, besonders bevorzugt zwischen 47°C und 53°C. Diese Temperaturbereiche haben sich als besonders effektiv sowohl zur Behandlung von Juckreiz, beispielsweise nach Insektenstichen oder als auch zur Behandlung von Herpeserkrankungen erwiesen.

Auch bei einer Anpassung der Behandlungsprogramme an unterschiedliche Anwendungen oder Nutzervorlieben, ist es daher besonders bevorzugt die Behandlungstemperaturen der einzelnen Behandlungsprogrammen aus den vorgenannten Bereichen zu wählen.

In einer weiteren Ausführungsform betragen die Behandlungstemperaturen zwischen von 42 °C - 56°C auf. Die Leistungen bezüglich der Behandlung von Juckreiz konnten durch den in dieser Ausführungsform bereitgestellten Temperaturbereich überraschend gesteigert werden.

In einer weiteren Ausführungsform betragen die Behandlungstemperaturen zwischen 42 °C - 53°C. So kann ein weiteres Mittel zur Behandlung von Juckreiz bereitgestellt werden.

In einer weiteren Ausführungsform betragen die Behandlungstemperaturen zwischen 43 °C - 48°C. Die Effektivität dieser Behandlungstemperaturen bei der Behandlung von Herpes ist überraschend groß.

In einer weiteren Ausführungsform betragen die Behandlungstemperaturen zwischen 47 °C - 53°C auf. Dieser Temperaturbereich hat sich als besonders wirksam und bei Probanden als beliebt herausgestellt. Hier kam es zu einem glücklichen Griff, denn aus einem weiten Parameterbereich wurde gerade der Bereich herausgegriffen, der besonders erfolgreich gegen Juckreiz wirkte.

In einer weiteren Ausführungsform wird die Behandlungstemperatur von 50 °C - 55 °C, 55° - 60 oder 60 °C - 65 °C auf. Diese Temperaturbereiche wurden entgegen dem Trend in der wissenschaftlichen Technik gewählt und können dennoch in einigen Probanden wirksame Linderung verschaffen.

In einer weiteren Ausführungsform wird die Behandlungstemperatur für eine Behandlungsdauer von 1 - 12 sec, bevorzugt 1-10 sec, 3 bis 5 sec, 4 bis 6 sec aufrechterhalten. Durch diese kurze und gleichzeitig besonders wirksame Behandlungsdauer kann die Effizienz der Vorrichtung gesteigert werden.

In einer weiteren bevorzugen Ausführungsform ist in einem ersten der auswählbaren Behandlungsprogramme eine Behandlungstemperatur zwischen 48°C und 53°C festgelegt, während in einem zweiten der auswählbaren Behandlungsprogramme eine Behandlungstemperatur zwischen 45°C und 48°C festgelegt ist.

Die Bereitstellung der mindestens zwei Behandlungsprogramme mit den vorgenannten Parametern erlaubt es für häufig auftretende Probandengruppen optimale Behandlungsprogramme bereitzustellen. So hat sich gezeigt, dass sich Probanden in Bezug auf die Behandlungstemperatur häufig in zwei Gruppen einteilen lassen. Eine Gruppe empfindet eine Behandlungstemperaturen von mehr als 48°C als zu schmerzhaft, während eine andere Gruppe gerade bei Temperaturen oberhalb von 48°C über eine besonders starke Linderung des Juckreizes berichten.

In einer weiteren bevorzugen Ausführungsform ist in einem ersten der auswählbaren Behandlungsprogramme einer Behandlungsdauer von 1 - 3 Sekunden, während in einem zweiten der auswählbaren Behandlungsprogramme einer Behandlungsdauer zwischen 3-12 Sekunden festgelegt ist. Ebenso hat sich gezeigt, dass sich in Bezug auf die Behandlungsdauer verschiedene Probanden zwei Bereiche als besonders bevorzugt präferierten. Für einige ist eine kurze Behandlungsdauer ggf. bei höheren Behandlungstemperatur angenehmer, während andere Probanden längere Behandlungsdauern ggf. bei niedrigen Behandlungstemperaturen als besonders effektiv beschreiben.

In einer bevorzugten Ausführungsform der Erfindung beträgt die Größe der Behandlungsfläche weniger als 1 cm², bevorzugt zwischen 20 und 80 mm².

Diese Größen der Behandlungsflächen haben sich als besonders vorteilhaft zur Behandlung von Juckreiz nach einem Insektenstich oder auch zur Behandlung von Herpeserkrankungen erwiesen.

Bei Herpeserkrankungen, insbesondere am Mund (sogenanntes Herpes *labialis*), ist eine maximale Größe der Behandlungsfläche von 60 mm² ideal, um alle möglichen betroffenen Stellen abzudecken. Insbesondere eine Behandlungsfläche zwischen 20 und 50 mm² ist geeignet, alle typischen betroffenen Hautpartien bei nur einmaligem Auflegen der Vorrichtung abzudecken. Des Weiteren kann eine Herpesbehandlungseinrichtung, welche eine solche Behandlungsfläche aufweist, besonders kompakt gehalten werden.

Zur Behandlung von Insektenstichen, insbesondere Mückenstichen ist die Größe der Behandlungsfläche bevorzugt zwischen 10 mm² und 100 mm² besonders bevorzugt zwischen 20 mm² und 60 mm². Diese Größen eignen sich um die gesamte betroffene Partie zielsicher zu behandeln, ohne nicht betroffene Hautstellen unnötig zu erhitzen.

Zudem sind derartige Vorrichtungsgrößen besonders kompakt und können der Größe eines Lippenstifts entsprechen. Ein solch kompaktes Gerät wird gern und bereitwillig dauerhaft am Körper oder in einer mitgeführten Tasche getragen, sodass eine Behandlung jederzeit vorgenommen werden kann. Dies steigert den Behandlungserfolg erheblich.

Die Behandlungsfläche ist bevorzugt rund, dies eignet sich besonders zur Behandlung von Herpes oder Insektenstichen, dessen betroffene Hautpartien oftmals annähernd runde Form aufweisen. Es können aber auch beliebige, dreidimensionale Formen, insbesondere konvexe Formen verwendet werden, welche sich für die Behandlung von Herpes oder Insektenstichen besonders eignen. Zum Beispiel kann die Form eines Lippenstifts verwendet werden, wobei der Benutzer bei der Behandlung zu einem leichten Aufdrücken der Vorrichtung animiert wird. Hierdurch kann ein psychologischer Effekt ausgelöst werden, der ein Wohlbefinden bei der Behandlung verstärkt. Auch kann die übertragene Wärmemenge verbessert werden. Es kann eine organische Form verwendet werden, welche sich zur Behandlung insbesondere der Lippen oder anderer Hautpartien besonders gut eignet.

In einer bevorzugten Ausführungsform heizt das Heizelement die Behandlungsfläche auf eine Behandlungstemperatur zwischen 42 °C und 56 °C, bevorzugt zwischen 45°C und 53°C, und besonders bevorzugt zwischen 47°C und 53°C auf und die Behandlungstemperatur wird für eine Behandlungsdauer von 1 sec bis 12 sec, bevorzugt 1 bis 10 sec aufrechterhalten. Die vorgenannten Bereiche für Behandlungsparameter eignen sich besonders für die Behandlung Juckreiz nach einem Insektenstich, beispielsweise einem Mückenstich.

Hierdurch können Schmerzen und weitere unangenehme Empfindungen wie bspw. ein Ziehen oder Juckreiz überraschend wirksam gelindert werden. Dies ist insbesondere der Fall nach Insektenstichen beispielsweise einem Mückenstich. Der Wärmeimpuls bewirkt eine Nervenstimulation, welche die subjektive Wahrnehmung von Schmerzen oder Juckreiz an den betreffenden Stellen stark reduziert. Die Wärmeübertragung führt überraschend zu einer Überlagerung der unangenehmen Empfindung durch andere temperaturabhängige Hautempfindungen. Entgegen konventionellen Methoden zur Behandlung von Juckreiz wird dabei zusätzlich eine Regulation der Schmerzrezeptoren anvisiert und durch die bevorzugte Wärmebehandlung die freien Nervenenden der C-Fasern aktiviert. Insbesondere wird die Erkenntnis über den Wirkmechanismus bei Juckreiz ausgenutzt, um durch eine Wärmebehandlung in überraschender Weise die Sinneswahrnehmung, welche durch die Fasern ausgelöst werden, zu regulieren.

Es wurde darüber hinaus erkannt, dass bei dieser Kombination eine besonders starke Überlagerung der unangenehmen Empfindungen erreicht werden kann, da in den betreffenden Hautpartien lokal die Thermo- und Capsaicinrezeptoren TRPV1 und TRPV2 zeitgleich aktiviert werden. Ein Fachmann würde auch unter Kenntnis der Literatur nicht davon ausgehen, dass gerade die Aktivierung dieser Rezeptoren eine besonders effektive Überlagerung der unangenehmen Empfindungen ermöglicht.

Die Behandlungstemperaturen zwischen 42 °C und 56 °C, bevorzugt zwischen 45°C und 53°C, und besonders bevorzugt zwischen 47°C und 53°C insbesondere im Zusammenhang mit eine Behandlungsdauer von 1 sec bis 12 sec, bevorzugt 1 bis 10 sec sind besonders bevorzugt bei einer Anwendungen gegen Juckreiz nach Insektenstichen, sodass diese besonders bevorzugt bei einer Behandlungsfläche von 10 mm² und 100 mm² besonders bevorzugt zwischen 20 mm² und 60 mm² zum Einsatz kommen.

In einer bevorzugten Ausführungsform heizt das Heizelement die Behandlungsfläche auf eine Behandlungstemperatur zwischen 42°C und 53°C, besonders bevorzugt zwischen 43°C und 48°C auf und die Behandlungstemperatur wird für eine Behandlungsdauer von 1 sec bis 12 sec, bevorzugt 1 bis 10 sec, besonders bevorzugt zwischen 2-5 sec aufrechterhalten. Die vorgenannten Bereiche für Behandlungsparameter eignen sich besonders für die Behandlung von Herpeserkrankungen.

Studien haben gezeigt, dass das Risiko einer Verbrennung bei einem Temperaturniveau von 44°C bis 51° mit jedem Grad Celsius um einen Faktor zwei zunimmt. Zudem wurde erkannt, dass eine Thermolabilität des DNA-bindenden Proteins ICP8 ausgenutzt werden kann, um die Replikation der Herpesvirus DNA wirksam zu unterbinden. Studien belegen eine Reduktion der Bindungsaktivität des Proteins an der Virus DNA um ca. 50% bei einer Temperatur von 45°C.

Einerseits sollte somit gegen Herpes eine möglichst hohe Temperatur für das Einbringen der Wärme gewählt werden, anderseits kann ein damit verbundener Schmerz dazu führen, dass die Behandlung vorzeitig abgebrochen wird und sich somit kein Erfolg einstellt.

Durch eine Auswahl der Temperaturbereiche von 43-48°C für die Behandlungsphase von 1-10 sec können sehr gute Ergebnisse in Bezug auf einen Rückgang der Herpesbläschen innerhalb von wenigen Tagen erreicht werden, ohne dass über stechenden Schmerz geklagt wird. Die Compliance und der Therapieerfolg sind hierfür durchweg gut.

Die vorgenannten Temperaturbereiche sind zudem besonders bevorzugt im Zusammenhang mit einer Behandlungsfläche von bevorzugt zwischen 10 mm² und 80 mm², besonders bevorzugt zwischen 20 mm² und 50 mm², welche bevorzugt bei Herpeserkrankungen ist.

In einer weiteren bevorzugten Ausführungsform beträgt die Größe der Behandlungsfläche zwischen 1 cm² und 18 cm², bevorzugt zwischen 6 und 9 cm².

Diese Ausführungsform eignet sich besonders zur Behandlung großflächig von Juckreiz betroffen Hautpartien wie diese im Falle von chronischem Pruritus, Allergien oder Quallenstichen.

Es wurde erkannt, dass mit einer größeren Behandlungsfläche von ca. 1 cm² bis 18 cm², bevorzugt mindestens 2 cm², mindestens 3 cm², mindestens 4 cm², besonders bevorzugt zwischen 6 cm² bis 9 cm² und ganz besonders bevorzugt zwischen 7 cm² und 9 cm² auf überraschender Weise auch großflächige Hautpartien, welche von Pruritus betroffen sind, behandelt werden können. So ist es beispielsweise möglich im Falle von Hautauschlägen durch bequemes und einfaches Auflegen der Behandlungsfläche auf die entsprechenden Hautpartien, das Juckempfinden durch die Hitzeübertragung in eine erträgliche Schmerzempfindung zu überführen. Sekundäre Schädigungen der Haut, beispielsweise Wundbildungen durch starkes Kratzen, können so wirksam vermieden werden. Mit Vorrichtungen des bekannten Standes der Technik wäre für eine Behandlung von großflächigen Hautpartien ein mehrmaliges Auflegen an unterschiedlichen Positionen notwendig. Aufgrund des Zeitversatzes kann hierdurch jedoch nicht derselbe Effekt erzielt werden kann.

Es kann auch bevorzugt sein, dass eine Behandlungsfläche von mindestens 6 cm² oder mindestens 7 cm² Größe Verwendung findet.

Äußere Reize chemischer, mechanischer oder physikalischer Natur, die Juckempfinden auslösen können, werden von drei unterschiedlichen Rezeptorzellen (Sinneszellen) wahrgenommen. Bei diesen Sinneszellen handelt es sich um so genannte offene Nervenendigungen, deren reizaufnehmende Strukturen in der Epidermis und der darunterliegenden Dermis lokalisiert sind, und deren Axone die Signale über wahrgenommene Reize ins Rückenmark weiterleiten. Bei diesen Sinneszellen sind die unmyelenisierten C-Fasern von besonderer Bedeutung. Deren rezeptive Strukturen befinden sich teilweise bis 0.1 mm unter der Hautoberfläche. Bei den C-Fasern unterscheidet man polymodale mechanisch und hitzesensitive Fasern und mechanisch insensitive C-Fasern, die aber auch durch Hitze stimuliert werden können. C-Fasern nehmen nicht nur pruritogene Reize war, sondern dienen auch als Nozizeptoren (Schmerzrezeptoren). In der Literatur wurde gezeigt, dass Hitzereize als Gegenreiz Juckempfinden unterdrücken können. Die einzelnen C-Fasern nehmen Reize eines bestimmten Areals der Haut wahr, wobei ein definiertes Hautareal von einer Sinneszelle innerviert wird. Diese Region wird als rezeptives Feld bezeichnet. Die rezeptiven Felder von C-Fasern können teilweise überlappend sein. Durch Untersuchungen am Menschen durch so genanntes Micromapping wurde überraschenderweise erkannt, dass die mechanisch insensitiven C-Fasern rezeptive Felder von bis zu 5 cm² Größe haben; die von mechanisch sensitiven C-Fasern sind etwas kleiner und bis zu 2 cm² groß. Überraschenderweise hat sich gezeigt, dass ab einer bevorzugten Größe der Behandlungsfläche von ungefähr 6 cm² oder 7 cm² erreicht werden kann, die rezeptiven Felder beider Typen von C-Fasern besonders gut anzusprechen. Bei der bevorzugten Behandlungsgröße zwischen 7 cm² und 18 cm² werden somit die rezeptiven Felder der unterschiedlichen C-Fasertypen überraschend gut abgedeckt und darüber hinaus wird der Effekt der horizontal abfließenden Wärme kompensiert. Somit kann ein Juckreiz, auch von betroffenen Hautpartien kleinerer Größe als der Behandlungsfläche, überraschender Weise sehr effektiv behandelt werden.

In einer bevorzugten Ausführungsform umfasst die Vorrichtung mindestens einen Energiespeicher.

Energiespeicher können beispielsweise durch mindestens einen Akku, eine Batterie und/oder einen Kondensator realisiert werden. Gemeint ist bevorzugt ein elektrischer Energiespeicher. Eine Batterie ist ein Energiespeicher, bevorzugt ein elektrochemischer Energiespeicher, dessen gespeicherte Energie nach dem Verbrauch bevorzugt nicht wieder aufgefüllt werden kann. Es können Zink-Mangan-Batterien, Alkali-Mangan-Batterien, Zinkchlorid-Batterien, Zink-Kohle-Batterien, Zink-Luft-Batterien, Quecksilberoxid-Zink-Batterien, Silberoxid-Zink-Batterien, Nickel-Oxyhydroxid-Batterien, Lithiumbatterien; Lithium-Eisensulfid-Batterien, Aluminium-Luft-Batterien, Biobatterien, z. B. auf Basis Magnesium/NaCI/Eisen + Molybdän + Wolfram und/oder Edison-Lalande-Elemente verwendet werden. Batterien können auch sogenannte Knopfzellen sein.

Ein Kondensator ist bevorzugt ein elektrisches Bauteil, welches elektrische Ladung in einem elektrischen Feld speichert.

Der Energiespeicher kann insbesondere mindestens ein Akku sein.

Akkus bzw. Akkumulatoren sind im Gegensatz zu Batterien bevorzugt wiederaufladbar. Akkus können beispielsweise Lithium-Ionen-Akkus, Lithium-Cobaltdioxid-Akkus, Lithium-Polymer-Akkus, Lithium-Mangan-Akkus, Lithium-Eisenphosphat-Akkus, Lithium-Eisen-Yttrium-Phosphat-Akkus, Lithium-Titanat-Akkus, Lithium-Metall-Polymer-Akkus, Lithium-Luft-Akkus, Lithium-Schwefel-Akkus, Natrium-Nickelchlorid-Hochtemperatur-Akkus, Natrium-Schwefel-Akkus, Natrium-Ionen-Akkus, Nickel-Cadmium-Akkus, Nickel-Eisen-Akkus, Nickel-Wasserstoff-Akkus, Nickel-Metallhydrid-Akkus, Nickel-Zink-Akkus, Bleiakkus, PTMA-Akkus, Rechargeable Alkaline Manganese Akkus, Zinn-Schwefel-Lithium-Akkus, Silber-Zink-Akkus, Vanadium-Redox-Akkus und/oder Zink-Brom-Akkus, Silizium-Luft-Akkus sein. Es können auch Lithium-Polymer und/oder ein Metall-hydrid-Akkus zum Einsatz kommen, welche besonders flexible und den Anwendungen angepasste Bauformen aufweisen und besonders leistungsstark sind.

Diese Ausführungsform der Vorrichtung hat den Vorteil, dass die Vorrichtung sowohl bei verbundenem Interface mit einem Mobilgerät, als auch autark mit elektrischer Energie versorgt ist.

Insbesondere die Ausführungsform umfassend mindestens ein Akku (oder eventuell auch ein Kondensator) kann bei verbundenem Interface mit einem Mobilgerät zudem über dieses mit elektrischer Energie versorgt und dabei aufgeladen werden.

Der Akku kann dabei so dimensioniert sein, dass er für mindestens eine Anwendung, bevorzugt mindestens 10, 20, 30, besonders bevorzugt mindestens 40 Anwendungen Energie bereitstellen kann. Hierdurch ist die Vorrichtung für eine Verwendung nicht auf eine Verbindung mit dem Mobilgerät angewiesen und besonders flexibel.

In einer bevorzugten Ausführungsform umfasst der Akku ein Festkörperakku, bevorzugt ein Lithium - Keramik - Akku. Dieser zeichnet sich durch eine besonders hohe Sicherheit aus und ist bevorzugt auch nach einer teilweisen Zerstörung gebrauchsfähig.

In allen vorstehend genannten Ausführungsformen kann die Vorrichtung kompakt gehalten werden und beispielsweise direkt per Schnittstelle mit dem Mobilgerät, z. B. einem Smartphone, gekoppelt werden. Ein Benutzer kann in diesem Fall die Vorrichtung durch sein Smartphone nutzen, beispielsweise indem er die Vorrichtung auf das Smartphone steckt, wobei eine mechanische Verbindung bevorzugt durch die Kopplung der Schnittstellen selber bewirkt wird, und das Smartphone zur Steuerung und/oder als Griff der Vorrichtung verwendet. Bei drahtlosen Schnittstellen kann die Vorrichtung selber so geformt sein, dass sie mechanisch, beispielsweise durch Aufstecken, mit dem Mobilgerät verbindbar ist und daher auch ohne kabelgebundene Schnittstelle eine Verbindung möglich ist.

Besonders bevorzugt ist es jedoch, wenn die Vorrichtung autark verwendet wird und diese einen eigenen, kompakten Energiespeicher umfasst. Es hat sich gezeigt, dass im Gegensatz zu einer unmittelbaren Kopplung an ein Mobilgerät eine verbesserte Handhabung erzielbar ist. So lässt eine betroffene Hautpartie mit Hilfe einer kompakten, aber autarken Vorrichtung deutlich leichter treffen, als mit einer Vorrichtung, welche an einem Mobilgerät aufgesetzt wird. Im letzteren Falle verhindert das Mobilgerät teilweise die Sicht, wohingegen im ersten Fall die Abmaße einer Vorrichtung so gewählt werden können, das die Vorrichtung besonders handlich in der Hand liegt, ohne die Sicht zu versperren.

In einer bevorzugten Ausführungsform der Erfindung das Interface zusätzlich zur Stromversorgung durch ein Mobilgerät konfiguriert ist.

Dafür ist bevorzugt, eine Energieübertragung vom Mobilgerät zur Vorrichtung über das Interface zu ermöglichen. Die Energieübertragung sieht insbesondere die Übertragung elektrischer Energie vor. Die Energie kann aber auch in anderer Form übertragen und dann von der Vorrichtung in elektrische Energie umgewandelt werden. Die Übertragung, insbesondere von elektrischer Energie, kann kabelgebunden stattfinden. In diesem Fall wird das Interface insbesondere gebildet durch eine Möglichkeit der Kabelverbindung zwischen Mobilgerät bzw. dessen Schnittstelle und der Schnittstelle der Vorrichtung.

Die Übertragung kann aber auch drahtlos stattfinden, wobei von Seiten der Vorrichtung das hierfür geeignete Interface bevorzugt aus einem Energiewandler besteht, der die drahtlos übertragene Energie in elektrische Energie umwandelt, welcher zur Stromversorgung der Vorrichtung verwendet werden kann. Die am Interface empfangene, übertragene elektrische Energie wird bevorzugt an die Elemente der Vorrichtung übertragen, welche elektrische Energie verbrauchen, wie beispielsweise das Heizelement oder die Regelvorrichtung. Diese Übertragung kann durch geeignete Verbindungen, z. B. durch Kabel, realisiert werden. Hierbei kann ein Energiespeicher zwischen- und/oder nachgeschaltet sein. Die Verwendung geeigneter Spannungsregler und/oder Laderegler zum Versorgen und/oder Laden des Energiespeichers und die elektrische Verschaltung einzelner Elemente der Vorrichtung mit der Schnittstelle ist ebenso bevorzugt und von einem Fachmann routinemäßig vornehmbar.

Insbesondere können auch die Übertragungen, von Energie und Daten parallel stattfinden. Einige Schnittstellen, wie zum Beispiel USB, sehen eine parallele Spannungs- und Datenübertragung vor. Auch bei einem drahtlosen Interface kann elektrische Energie und/oder Daten, bevorzugt parallel, übertragen werden, beispielsweise durch Induktionsspulen oder durch elektromagnetische Strahlung, welche durch Solarzellen und/oder Photodioden in elektrische Spannung umgewandelt wird.

In einer bevorzugten Ausführungsform ist die Vorrichtung konfiguriert für eine Stromversorgung und/oder ein Laden des Energiespeichers durch drahtlose Energieübertragung, bevorzugt über das Interface.

Bei der drahtlosen Energieübertragung wird die elektrische Energie bevorzugt übertragen, ohne dass zwischen der zu ladenden Vorrichtung und dem Mobilgerät eine Verbindung über ein elektrisches Kabel besteht. Die elektrische Energie kann dabei beispielsweise durch elektromagnetische Felder übertragen werden. Dafür kann bevorzugt induktive Kopplung durch jeweils mindestens eine Spule im Mobilgerät und in der Vorrichtung verwendet werden. Es kann ebenso vorteilhaft sein, Vorrichtung und Mobilgerät durch jeweils mindestens eine Kondensatorplatte kapazitiv zu koppeln. Auch über weite Distanzen kann durch sogenannte Fernfeldübertragung kann bevorzugt elektrische Energie übertragen werden, die in den zwischen Sender und Empfänger transmittierten elektromagnetischen Wellen steckt.

Ebenso kann durch das Mobilgerät emittiertes Licht Energie übertragen werden, die in mindestens einer Solarzelle der Vorrichtung in elektrische Spannung umgewandelt wird. Es kann auch bevorzugt sein, elektrische Energie zwischen leitenden Oberflächen des Mobilgeräts und der Vorrichtung über den zwischen diesen Oberflächen bestehenden Kontakt direkt zu übertragen. Die übertragene Energie kann dabei direkt zum Aufheizen des Heizelements oder aber bevorzugt zum Laden eines in der Vorrichtung integrierten Akkus verwendet werden.

Ein Fachmann wüsste, dass er die Vorrichtung mit hierfür geeigneten Elementen wie z. B. mindestens einer Induktionsspule, einer Kondensatorplatte, einer Antenne, einer Solarzelle, einer Photodiode, einem Laderegler und/oder einem Spannungsregler ausstatten müsste und wie er die geeigneten Elemente verschalten müsste, um die drahtlose Energieübertragung zu realisieren.

Durch eine drahtlose Übertragung können lästige Kabel zwischen Vorrichtung und Mobilgerät vermieden werden.

In einer bevorzugten Ausführungsform ist die Vorrichtung konfiguriert für eine Stromversorgung und/oder ein Laden des Energiespeichers durch ein Kabel, insbesondere über die Schnittstelle. In dieser Ausführungsform umfasst die Vorrichtung, insbesondere die Schnittstelle, ein Kabel bzw. einen Stecker und/oder eine Buchse zur Verbindung mit einem Kabel. Des Weiteren ist bevorzugt, die elektrische Energie innerhalb der Vorrichtung an geeignete Elemente, insbesondere an den Energiespeicher durch beispielsweise Verbindungen weitergeleitet wird. Zwischengeschaltet ist vorteilhafterweise ein Spannungsregler und/oder Ladungsregler. Einem Fachmann sind geeignete Elemente und deren Verschaltungen bekannt. Eine Übertragung von elektrischer Energie auf diese Art ist besonders robust, energieeffizient und kostengünstig.

In einer bevorzugten Ausführungsform ist ein Interface ausgesucht ist aus der Gruppe umfassend: Bluetooth, Lightning, Klinkenstecker, Koaxialstecker, Apple 30-pin dock connector, ASUS Media Bus proprietary, CAMAC, EISA, ISA, LPC, MBus, MCA, Multibus for industrial systems, NuBus oder IEEE 1196, OPTi local bus, PCI, ATA, PATA, IDE, EIDE, ATAPI, S-100 bus oder IEEE 696, SBus oder IEEE 1496, SS-50 Bus, Runway bus, GSC/HSC, Precision Bus, STEbus, STD Bus, Unibus, Q-Bus, VLB oder VL-bus, VMEbus, PC/104, PC/104-Plus, PCI-104, PCI/104-Express, PCl/104, Zorro II and Zorro III, 1-Wire, HyperTransport, I²C, PCle, SATA, SPI bus, UNI/O, SMBus, IrDA, WLAN, ZigBee, NFC, Wibree, WiMAX, IrDA, optischer Richtfunk, eBus, USB, Micro USB, Type C und/oder FireWire.

Durch Bereitstellung eines Interface ausgesucht aus dieser Gruppe ist eine hohe Flexibilität bezüglich des verwendeten Mobilgeräts und der zur Verfügung stehenden Schnittstelle gegeben. Des Weiteren haben diese Schnittstellen ihre Tauglichkeit für eine Vielzahl verschiedenster Anwendung unter Beweis gestellt. Dabei ist einem Fachmann bekannt, wie er eine solche Schnittstelle konstruieren müsste. Beispielsweise wüsste ein Fachmann, dass er für eine Lightning Schnittstelle eine geeignete Buchse und/oder einen geeigneten Stecker verbauen müsste, ebenso wüsste er, dass er geeignete Prozessoren, z. B. Host-Controller verwenden müsste. Ihm wäre ebenso bekannt, wie er die elektrischen Elemente der Vorrichtung mit der Schnittstelle verschalten müsste.

Bei z. B. einer WLAN Schnittstelle wüsste der Fachmann ebenso, dass er mindestens eine geeignete Antenne zum Versenden und/oder Empfangen von Signalen in die Vorrichtung verbauen müsste. Auch die Auswahl geeigneter Steuerungsprozessoren, Datenwandler und/oder Controller sowie eine Verschaltung der Schnittstelle innerhalb der Vorrichtung ist für den Fachmann routinemäßig durchführbar.

Dabei kann es bevorzugt sein, die Schnittstelle zu verwenden, um die Vorrichtung zur Stromversorgung und/oder zum Aufladen direkt mit einer Steckdose zu verbinden. In einer bevorzugten Ausführungsform ist es beispielsweise möglich, mittels handelsüblichem USB-Ladegerät elektrische Energie direkt aus der Steckdose zu beziehen.

In einer bevorzugten Ausführungsform ist das Interface zusätzlich für einen Datenaustausch mit einem Mobilgerät konfiguriert ist.

Daten sind bevorzugt Informationen, die von einem elektronischen Datenverarbeitungsgerät ausgegeben, empfangen und/oder verarbeitet werden können. Ein Datenverarbeitungsgerät ist bevorzugt eine Vorrichtung ausgesucht aus der Gruppe Computer, Mikrocomputer, Prozessor, Mikroprozessor, integrierter Schaltkreis, Smartphone, sonstige Mobilgeräte und/oder Regelvorrichtung. Bevorzugt sind Daten in digitalem Format, insbesondere in Bits. Auch analoge Daten sind jedoch vorstellbar. Ebenso können Daten bevorzugt in einem Speicher oder auf einem Speichermedium gespeichert werden.

Eine Datenübertragung mit einem Mobilgerät bezeichnet bevorzugt den Austausch von Daten mit dem Mobilgerät. Dabei können Daten bevorzugt von der Vorrichtung empfangen und/oder gesendet werden. Ebenso können Daten bevorzugt von der Vorrichtung gespeichert und/oder verarbeitet werden.

Um für eine Datenübertragung über die Schnittstelle konfiguriert zu sein, sollte bevorzugt über die Schnittstelle eine Datenleitung, bevorzugt mehrere parallele Datenleitungen zwischen Vorrichtung und Mobilgerät über die Schnittstelle eingerichtet sein. Das bedeutet, dass über Datenleitungen eine Übertragung zwischen Mobilgerät und Schnittstelle sowie zwischen Schnittstelle und weiteren elektrischen Komponenten der Vorrichtung möglich ist. Die Übertragung zwischen Mobilgerät und Schnittstelle kann bevorzugt über eine Schnittstelle des Mobilgeräts erfolgen, welche je nach Schnittstelle beispielsweise kabelgebunden und/oder drahtlos erfolgen kann, wie bereits obenstehend geschildert wurde. Die Übertragung zwischen Schnittstelle der Vorrichtung und den weiteren Komponenten kann beispielsweise durch physische Signalleitungen in Form von Kabeln realisiert werden, welche die Übertragung elektrischer Signale ermöglichen.

Dabei kann es bevorzugt sein, dass mindestens eines der Elemente aus Mobilgerät, Schnittstelle des Mobilgeräts, Schnittstelle der Vorrichtung und elektrischen Komponenten der Vorrichtung ein unterschiedliches Datenformat verwendet als mindestens eines der übrigen Elemente aus dieser Gruppe. Daher kann es bevorzugt sein, dass an den Verbindungsstellen dieser Elemente, welche unterschiedliche Formate verwenden, mindestens ein Datenwandler vorliegt, der die Daten in Übertragungsrichtung in ein für das folgende Element geeignetes Datenformat überträgt. Sofern solch ein Datenwandler der Schnittstelle der Vorrichtung zugeordnet ist und/oder in Richtung der Komponenten der Vorrichtung dieser folgt, ist dieser mindestens eine Datenwandler bevorzugt Teil der Vorrichtung.

Zum Senden von Daten umfasst die Vorrichtung und/oder deren Schnittstelle bevorzugt mindestens eine Datensendeeinrichtung. Diese kann Daten der Vorrichtung über die Schnittstelle in Richtung Mobilgerät übertragen. Die Daten können dabei bevorzugt von einer in die Vorrichtung integrierten Regelvorrichtung generiert werden und/oder auf einem Datenspeicher der Vorrichtung vorliegen. Bevorzugt kann die Datensendeeinrichtung auch als Datenwandler agieren bzw. einen solchen umfassen. Einem Fachmann sind geeignete Einrichtungen bekannt und/oder er kann sie routinemäßig einrichten.

Zum Empfangen von Daten umfasst die Vorrichtung bevorzugt mindestens eine Datenempfangseinheit. Diese kann vom Mobilgerät kommende Daten über die Schnittstelle empfangen, diese gegebenenfalls in ein geeignetes Datenformat umwandeln und beispielsweise an eine Regelvorrichtung zur Weiterverarbeitung und/oder ein Speichermedium zum Speichern in geeigneter Weise weitergeben. Ein Fachmann kennt Möglichkeiten, geeignete Einheiten routinemäßig zu realisieren.

Es kann auch bevorzugt sein, dass Datensende- und empfangseinheit in einer gemeinsamen Einheit kombiniert sind.

Wie bereits vorstehend beschrieben, umfasst die Vorrichtung bevorzugt einen Speicher, welcher bei der Datenübertragung genutzt werden kann. Dabei kann es sich beispielsweise um einen Speicher ausgesucht aus der Gruppe umfassend Festkörperspeicher, RAM-Speicher, ROM-Speicher, EPROM-Speicher, EEPROM-Speicher, Flash-Speicher und/oder um andere Speichertechnologien handeln.

Die Daten können dabei parallel und/oder seriell übertragen werden. Bei einer parallelen Übertragung können mehrere digitale Informationseinheiten, sogenannte Bits, gleichzeitig übertragen werden.

Besonders bevorzugt ist es, dass die Daten von der Vorrichtung auf das Mobilgerät übertragen werden. Bei den Daten kann es sich beispielsweise um eine Angabe des derzeit ausgewählten Behandlungsprogramme sowie der festgelegten Behandlungsparameter, wie die Behandlungstemperatur, die Behandlungsdauer und/oder eine zeitliche Abfolge von anzuwendenden Behandlungstemperaturen handeln.

Bevorzugt kann das ausgewählte Behandlungsprogramm am Mobilgerät angezeigt werden, sodass der Nutzer stets ein Feedback darüber hat, ob die Auswahl korrekt erfolgt ist.

Auch können die Daten Messwerte der Vorrichtung beispielsweise eines Temperatursensors umfassen, wodurch in Echtzeit am Mobilgeräte der Behandlungsverlauf und das Erhitzen der Behandlungsfläche nachvollzogen werden kann. Die Funktionalität erfreut sich bei Probanden besonderer Beliebtheit und steigert in besonderem Maße die Nutzer Akzeptanz.

Die von der Vorrichtung übertragenen Daten können bevorzugt auch mit Nutzerdaten verknüpft werden. Hierbei können auch weitere relevante Metadaten generiert werden, die beispielsweise Behandlungserfolg, Heilungsverlauf, Häufigkeit der Anwendung usw. zum Inhalt haben.

Zusätzlich kann das Mobilgerät, welches beispielsweise ein Smartphone ist, weitere Daten generieren, wie beispielsweise GPS-Positionsdaten, Zeitstempel, Fotos von Juckreiz betroffenen Hautpartien etc. Diese können alle miteinander verknüpft und/oder korreliert werden, wodurch statistisch relevante Daten erzeugt werden können, die zum Beispiel erfolgreiche Behandlungstherapien betreffen oder aber einen geographisch und/oder zeitlich begrenztes Auftreten einer Insektenplage.

In einer bevorzugten Ausführungsform ist das Mobilgerät ausgesucht aus der Gruppe umfassend PC, Laptop, Desktop-PC, Mobiltelefon, Smartphone, Tablet-Computer, Personal Digital Assistant (PDA), Notebook, Subnotebook, Walkman, Discman, MP3-Player, Taschenfernseher (tragbare Fernsehgeräte), E-Book-Lesegerät, tragbares Ausgabegerät für elektronische Medien, GPS-Gerät, tragbares Schnittstellengerät der Satellitenkommunikation, Handheld, Pocket Computer (Taschencomputer), Mobilcomputer, Fotoapparat, Videokamera, Armbanduhr, Taschenrechner, Fernseher, MacBook, iPhone, iPad, iPod, iMac, Mac mini, Mac Pro, Smartwatch und/oder Powerbank.

Diese Mobilgeräte haben sich zur Verwendung mit der Vorrichtung als besonders praktisch herausgestellt. Insbesondere Mobilgeräte, welche eine hohe eigene Rechenleistung und eine hohe Flexibilität bezüglich ihrer Anwendung aufweisen, können in Verbindung mit der Vorrichtung ein besonders leistungsfähiges System bilden. Solche Geräte werden beispielsweise von Smartphones repräsentiert. Bei diesen können durch auf die Vorrichtung individuell zugeschnittene Anwendungsprogramme (sogenannte Apps) überraschende Vorteile erzielt werden. Beispielsweise können durch vernetzte Benutzerdaten statistisch relevante Aussagen zu erfolgversprechenden Behandlung getroffen werden, welche bei einer genügend hohen Anzahl an ausgewerteten Daten weit über das Maß der Aussagekraft einer bestimmten medizinischen Studie hinausgehen. So können durch Vorhersagen von Insektenplagen aufgrund aus der Vergangenheit gewonnene Nutzerdaten synergistische Effekte erzielt werden, da durch die Vorrichtung nun nicht nur Insektenstiche behandelt werden können, sondern auch verhindert werden können. Auch eine verbesserte medizinische Behandlung von Juckreiz aufgrund einer Aufwertung individueller Nutzerdaten bei einem Facharzt ist möglich.

In einer bevorzugten Ausführungsform der Erfindung umfasst die Vorrichtung mindestens einen Temperatursensor zur Messung der Temperatur der Behandlungsfläche, wobei die Regelvorrichtung basierend auf den Messdaten des Temperatursensors das mindestens eine Heizelement reguliert.

Im Sinne der Erfindung ist ein Temperatursensor bevorzugt ein elektrisches oder elektronisches Bauelement, welches in Abhängigkeit der Temperatur am Sensor ein elektrisches Signal generiert. Im Stand der Technik sind eine Vielzahl von Temperatursensoren bekannt, wie beispielsweise Halbleiter-Temperatursensoren, Widerstandstemperatursensoren, pyroelektrische Materialien, Thermoelemente oder Schwingquarze.

Die Regelvorrichtung ist weiterhin bevorzugt derart konfiguriert, dass diese die Messwerte der Temperatursensoren aufnehmen und auswerten kann, um eine Regulation der Heizplatten zu bewirken. Die Regulation der Heizplatten kann bevorzugt mithilfe des Anliegens eines elektrischen Stroms oder einer Spannung erfolgen.

Es ist besonders bevorzugt, dass der Temperatursensor die Temperatur der Behandlungsfläche direkt misst, d.h. dass der Temperatursensor in Kontakt mit der Behandlungsfläche ist, wobei sich der Temperatursensor sowohl auf der inneren Seite der Behandlungsfläche, als auch auf der äußeren Seite der Behandlungsfläche befinden kann oder aber in die Behandlungsfläche implementiert ist. Es kann aber auch bevorzugt sein, dass der Temperatursensor nicht die Behandlungsfläche direkt, sondern die Heizelemente oder einen Materialpunkt zwischen den Heizelementen und der Behandlungsfläche kontaktiert und überwacht. Im Falle von mehreren Heizelementen, welche die Behandlungsfläche erhitzen, kann es beispielsweise auch bevorzugt sein den Temperatursensor zwischen den Heizelementen zu platzieren. Aus den Messdaten für die Temperatur über die Heizelemente oder einem Messpunkt in einer bestimmten Distanz zur Behandlungsfläche kann ebenfalls auf die Temperatur der Behandlungsfläche geschlossen werden. Im Sinne der Erfindung ist es bevorzugt, dass die Temperatur der Behandlungsfläche die durchschnittliche Temperatur der Behandlungsfläche meint. Besonders bevorzugt meint die Behandlungstemperatur die durchschnittliche Temperatur der Außenseite der Behandlungsfläche, während diese während der Anwendung des Gerätes eine Haut kontaktiert.

Eine Auswertung der Temperatur der Behandlungsfläche erlaubt eine besonders präzise Regulation des mindestens einen Heizelementes, um eine optimale Temperaturverteilung auf der Behandlungsfläche und somit Wärmeübertragung an die zu behandelnden Hautpartien sicherzustellen. Insbesondere im Hinblick auf die vielfältigen Anwendungsmöglichkeiten der Vorrichtung zur Behandlung verschiedener Erkrankungen, welche mit Juckreiz einhergehen können, ist eine temperaturbasierte Feedback-Regulation mit Hilfe der Regelvorrichtung geeignet, um eine zuverlässige hyperthermische Behandlung mit optimalen Temperaturwerten durchzuführen. Eine solche Vorrichtung zur Steuerung der Behandlungstemperatur ist besonders einfach, robusten und kostengünstig.

In einer weiteren bevorzugten Ausführungsform umfasst die Vorrichtung ein wasserdichtes Gehäuse. Das Gehäuse stellt bevorzugt eine äußere Ummantelung der Vorrichtung dar, sodass diese insbesondere die Regelvorrichtung und anderen elektronische Komponenten umschließt.

In der bevorzugten Ausführungsform ist das Gehäuse derart ausgestaltet, dass sämtliche Gehäusedurchbrüche, Bedienelemente, Schnittstellen, Gehäusetrennungen wasserdicht, bzw. unempfindlich gegenüber Wasser sind. Beispielsweise können zu diesem Zweck Dichtungsringe oder geeignete Dichtelemente, zum Beispiel aus Elastomeren, eingesetzt werden. Der Fachmann kennt jedoch viele weitere technische Möglichkeiten, um ein Gehäuse wasserdicht zu konstruieren. Die wasserdichte Ausgestaltung des Gehäuses stellt ein (zusätzliches) Sicherheitselement dar, da hierdurch Schäden an der Regelvorrichtung oder anderen elektronische Komponenten aufgrund von eintretenden Flüssigkeiten wirksam vermieden werden können. Auch führt das wasserdichte Gehäuse zur Vermeidung von Korrosion und somit zu einer verlängerten Lebensdauer der Vorrichtung. Insbesondere in Verbindung mit der Verwendung von Schutzlack kann die Sicherheit synergistisch erhöht werden. Dies spielt insbesondere bei Desinfektionsvorgängen der Vorrichtung und vor allem der Behandlungsfläche eine Rolle. Somit kann die Vorrichtung sehr einfach und fehlerfrei gründlich desinfiziert werden, indem bevorzugt die ganze Vorrichtung in eine Desinfektionsflüssigkeit getaucht bzw. verbracht wird und dort für eine gewisse Mindestdauer verbleibt.

In weiteren bevorzugten Ausführungsformen umfasst die Vorrichtung weitere Sicherheitselemente, welche die Temperatur der Behandlungsfläche kontrollieren.

In einer bevorzugten Ausführungsform umfasst Vorrichtung einen hardwareimplementierter Temperaturwächter die Maximaltemperatur der Behandlungsfläche reversibel begrenzt und eine Schmelzsicherung bei Kurzschluss oder ungeregeltem Durchheizen die Vorrichtung abschaltet.

Die Maximaltemperatur bezeichnet bevorzugt jene Temperatur, welche die Behandlungsfläche maximal während der Behandlungsphase erreicht. Der hardwareimplementierte Temperaturwächter erlaubt vorteilhafterweise eine Sicherstellung, dass die Maximaltemperatur nicht überschritten wird.

Der "hardwareimplementierte Temperaturwächter" bezeichnet bevorzugt ein Temperaturkontrollsystem für die Behandlungsoberfläche, welches hardwarebasiert die Stromversorgung der Heizelemente für die Behandlungsfläche abschalten kann. Insbesondere erlaubt der "hardwareimplementierte Temperaturwächter" bevorzugt, dass eine Abschaltung der Stromversorgung der Heizelemente bei Überschreitung der Maximaltemperatur unabhängig von der Regulation der Heizelemente durch die Regelvorrichtung, also z.B. dem Mikroprozessor erfolgt.

Sollte zur Regulation der Heizelemente beispielsweise auf der Regelvorrichtung eine Firmware installiert vorliegen, so ist es bevorzugt, dass der hardwareimplementierte Temperaturwächter auch bei einem Ausfall oder einem fehlerhaften Ausführen der Firmware die Maximaltemperatur der Behandlungsfläche zuverlässig begrenzt. Hierdurch kann durch einfache konstruktive Mittel besonders effektiv sichergestellt werden, dass die Behandlungsfläche der Vorrichtung eine Maximaltemperatur nicht überschreitet. Selbst unter Auftreten von Fehlsteuerungen in der Regelvorrichtung, beispielsweise nach dem Eintreten von Flüssigkeiten, kann aufgrund des hardwarebasierten Temperaturwächters vorteilhafterweise jederzeit gewährleistet werden, dass die Behandlungsfläche eine Maximaltemperatur nicht überschreitet. Durch dieses zusätzliche technische Element zur Temperaturüberwachung ist es möglich einen ausgezeichneten Sicherheitsstandard zu wahren, ohne mit der Funktionsweise der Vorrichtung zur hyperthermischen Behandlung zu interferieren.

Beispielsweise kann als Maximaltemperatur ein Wert zwischen 54 °C und 58 °C, bevorzugt ungefähr 56 °C gewählt werden. In dem Fall kann die Vorrichtung einen hardwareimplementierten Temperaturwächter umfassen, welcher die Maximaltemperatur der Behandlungsfläche auf einen Wert zwischen 54 °C und 58 °C bevorzugt ungefähr 56 °C begrenzt.

Im Sinne der Erfindung bezeichnen Angaben wie ungefähr, ca., nahezu oder synonyme Begriffe bevorzugt eine Toleranzspanne von weniger als ± 10%, bevorzugt weniger als ± 5% besonders bevorzugt weniger als ± 1%.

Eine solche Maximaltemperatur ist besonders bevorzugt im Zusammenhang mit einer Ausführungsform in welcher die auf der Regelvorrichtung gespeicherten Behandlungstemperaturen beispielsweise zwischen 45°C und 53°C liegen. Im Regelfall wird die Temperatur somit entsprechend der Auswahl des Behandlungsprogrammes auf eine bestimmte Behandlungstemperatur geführt, welche jedoch 53°C nicht überschreiten sollte. Falls aufgrund eines Ausfalls der Regelvorrichtung dies dennoch geschieht kann der hardware-implementierte Temperaturwächter zuverlässig sicherstellen, dass die Maximaltemperatur von beispielsweise 56°C nicht überschritten wird.

Auch andere, geeignete Maximaltemperaturen, z. B zwischen 53°C und 56 °C oder niedriger können je nach den vorgesehenen Behandlungsprogrammen bevorzugt sein.

Durch einen hardwareimplementierten Temperaturwächter kann durch einfache konstruktive Mittel besonders effektiv sichergestellt werden, dass die Behandlungsfläche der Vorrichtung eine Maximaltemperatur nicht überschreitet. Selbst unter Auftreten von Fehlsteuerungen in der Regelvorrichtung, beispielsweise nach dem Eintreten von Flüssigkeiten, kann aufgrund des hardwarebasierten Temperaturwächters vorteilhafterweise jederzeit gewährleistet werden, dass die Behandlungsfläche eine Maximaltemperatur (beispielsweise von einem Wert zwischen 54 °C und 58 °C bevorzugt von ungefähr 56 °C)nicht überschreitet. Durch dieses zusätzliche technische Element zur Temperaturüberwachung ist es möglich einen ausgezeichneten Sicherheitsstandard zu wahren, ohne mit der Funktionsweise der Vorrichtung zur hyperthermischen Behandlung zu interferieren.

Als weiteres Sicherheitselement kann die Vorrichtung eine Schmelzsicherung aufweisen, welche bei einem Kurzschluss der Vorrichtung oder ungeregeltem Durchheizen der Vorrichtung die Stromversorgung zur Vorrichtung unterbricht. Im Sinne der Erfindung wird unter einer Schmelzsicherung bevorzugt eine Überstromschutzeinrichtung verstanden, bei welcher beispielsweise durch das Abschmelzen eines Schmelzleiters ein Stromkreis unterbrochen werden kann, sobald die Stromstärke einen Grenzwert über eine zu bestimmende Zeit überschreitet. Es ist bevorzugt, dass die Schmelzsicherung in der Vorrichtung zwischen der Einspeisung der Versorgungsspannung in die Vorrichtung und der Vorrichtung selbst vorliegt. Sollte es zu einem Störfall kommen, welcher dadurch gekennzeichnet ist, dass ein unkontrollierter hoher Strom von der Versorgungseinspeisung in die Vorrichtung fließt, schaltet die Schmelzsicherung vorteilhafterweise die komplette Spannungsversorgung der Vorrichtung ab. Eine Schmelzsicherung bietet einen ausreichend schnellen und extrem zuverlässigen Schutz.

Es hat sich gezeigt, dass selbst unter fehlerfreier Konstruktion der Vorrichtung und dem Bereitstellen eines hardwareimplementierten Temperaturwächters es nicht ausgeschlossen werden kann, dass es aufgrund einer fehlerhaften Bedienung oder Schädigung der Vorrichtung, in äußerst seltenen Fällen zu einem Durchheizen der Heizelemente kommt. Unter dem Durchheizen der Heizelemente wird im Sinne der Erfindung bevorzugt verstanden, dass die Temperatur der Heizelemente unkontrolliert, d.h. nicht durch eine temperaturbasierte Regelung mithilfe der Regelvorrichtung steigt. Falls in diesen Störfällen der hardwareimplementierte Temperaturwächter versagt, kann die Behandlungsfläche unkontrolliert auf Temperaturen weit über der gewünschten Maximaltemperatur ansteigen, beispielsweise auf Temperaturen von weit über 65 °C.

Obwohl ein solches fehlerhaftes Durchheizen äußerst selten auftritt, kann dies zu Schädigungen bei den Probanden führen. Dies liegt insbesondere daran, dass die hyperthermisch zu behandelnden Hautpartien, wie die Lippen, zumeist besonders empfindlich sind und beispielsweise durch eine Rötung, Schwellung oder gar Wundbildung gekennzeichnet sind. Eine deutlich erhöhte Temperatur über 65° kann lokal an diesen Stellen in besonderem Maße zu starken Schmerzen und Hautverbrennungen führen.

Im Hinblick auf die besonderen Umstände der Nutzung der Vorrichtung und damit verbundenen Sicherheitsanforderungen ist die genannte Schmelzsicherung besonders vorteilhaft, um noch für den unwahrscheinlichsten Fall einer Störung eine Abschaltung des Heizens der Behandlungsfläche gewährleisten zu können. So wird mithilfe der Schmelzsicherung unabhängig von jeglicher Temperaturmessung, aufgrund z.B. fehlerhafter Temperatursensoren, ein übermäßiges Heizen der Behandlungsfläche unterbunden. Vorteilhafterweise stellt Stromversorgung der Vorrichtung eine zentrale Regulationsschnittstelle dar, welche höchsten Sicherheitsanforderungen genügt. Durch eine Integration der Schmelzsicherung in den Stromfluss zur Versorgung der Vorrichtung kann sichergestellt werden, dass ein maximaler Versorgungsstrom während einer bestimmten Zeit nicht überschritten wird. Da ein Durchheizen und ein unkontrolliertes Heizen der Heizelemente über die gewünschte Temperatur mit einem erhöhten Stromfluss zusammenhängt, kann hierdurch auf besonders zuverlässige Weise ein Überhitzen der Behandlungsfläche vermieden werden. Insbesondere kann durch die Stromkontrolle sehr schnell reagiert werden, bevor der Strom so lange wirkt, dass er eine seiner Stärke entsprechende Temperatur erzeugt. Ein rein an der Temperatur orientierter letztinstanzlicher Sicherheitsmechanismus könnte außerdem aufgrund von thermischen Trägheiten der beteiligten Bauteile nicht schnell genug sein.

Besonders vorteilhaft ist die kombinierte Verwendung eines hardwareimplementierten Temperaturwächters und einer Schmelzsicherung.

So ist es ein Nachteil der Schmelzsicherung, dass diese nach dem einmaligen Auslösen eine permanente Abkopplung der Versorgungsspannung von der Vorrichtung zur Folge hat. Eine erneute Inbetriebnahme der Vorrichtung nach dem Auslösen der Schmelzsicherung bedarf der Reparatur durch einen Techniker, beispielsweise den Austausch der Schmelzsicherung. Im Hinblick auf die Kosten ist die Vorrichtung in der Regel bei einem Auslösen der Schmelzsicherung unbrauchbar geworden.

Vorteilhafterweise ist der hardwareimplementierte Temperaturwächter jedoch so eingestellt, dass es nicht zu einem permanenten Abschalten der Stromversorgung der Vorrichtung kommen muss. Vielmehr ist der hardwareimplementierte Temperaturwächter derart konzipiert, dass wenn die Temperatur der Behandlungsfläche eine Maximaltemperatur überschreitet, während des Zeitraumes des Überschreitens die Stromversorgung der Heizelemente unterbrochen wird. Die Stromunterbrechung durch den hardwareimplementierten Temperaturwächter ist somit vorteilhafterweise reversibel, d.h. sobald die Temperatur der Behandlungsfläche wieder unter die Maximaltemperatur fällt, können die Heizelemente wieder heizen.

So kann auch nach einem einmaligen Auftreten einer Fehlsteuerung die bestimmungsgemäße Benutzung der Vorrichtung fortgeführt werden. Der Benutzer würde gegebenenfalls nichts von der Fehlsteuerung bemerken, da durch die gewählte Maximaltemperatur, die Effektivität und die Unabhängigkeit des hardwareimplementierten Temperaturwächters keine für den Benutzer als unangenehm empfundenen Temperaturen entstehen und die Vorrichtung bei der nächsten Benutzung im Falle einer einmaligen Fehlsteuerung wieder einwandfrei funktioniert könnte.

Die Kombination der Sicherheitsmerkmale eines hardwareimplementierten Temperaturwächters mit einer Schmelzsicherung erlauben eine überraschend zuverlässige Kontrolle der Temperatur mit möglichst wirtschaftlichen Mitteln aufgrund gestaffelter Sicherheitsschranken. Insbesondere bei einer Vorrichtung, die mit einem Mobilgerät verbunden wird, ist eine Kombination von weiteren Sicherheitsschranken sinnvoll.

Ein weiterer synergistischer Effekt durch die Kombination der Sicherheitsmerkmale eines hardwareimplementierten Temperaturwächters mit einer Schmelzsicherung kann darin gesehen werden, dass beispielsweise ein zwar unwahrscheinliches, jedoch mögliches einmaliges Versagen der Regelvorrichtung durch den hardwareimplementierten Temperaturwächter reversibel abgefangen wird. Sollte jedoch ein äußerst unwahrscheinliches größeres Problem auftreten, welches den hardwareimplementierten Temperaturwächter umfasst, so tritt die Schmelzsicherung als letztinstanzliche Absicherung in Aktion. Da diese jedoch irreversibel ist, kann keine unter diesen Umständen gefährliche Weiterbenutzung durch den Benutzer stattfinden, sondern es wird ein Gang zum Techniker bzw. zum Fachhandel veranlasst.

Bevorzugt erfolgt bereits mithilfe der Regelvorrichtung eine Temperierung der Behandlungsfläche. Falls die Regelvorrichtung aufgrund z.B. einer fehlerhaften Elektronik versagt, erlaubt der hardwareimplementierte Temperaturwächter ein Abschalten der Heizelemente unabhängig von der Regelvorrichtung. Selbst bei einer solchen Störung der Regelvorrichtung würde eine Schmelzsicherung nicht ausgelöst. Nur in dem äußerst seltenen Fall, dass sowohl die Regelvorrichtung, als auch der hardwareimplementierte Temperaturwächter versagt, beispielsweise bei einer Beschädigung der entsprechenden Bauelemente, gewährleistet die Schmelzsicherung ein finales Kontrollelement. Kommt es zu einem erhöhten Strombedarf der Heizelemente aufgrund eines starken Erhitzens, schaltet die Schmelzsicherung die gesamte Stromversorgung der Vorrichtung ab. Durch diese Staffelung der Sicherheitsmechanismen kann eine einmalige Störung der Regelvorrichtung äußerst sicher abgefangen werden. Der hardwareimplementierte Temperaturwächter schreitet unbemerkt und schnell ein, ohne die Benutzbarkeit der Vorrichtung zu beeinflussen. Durch die nachgeschaltete Schmelzsicherung kann ein noch höheres Sicherheitslevel erreicht werden, so dass dem Benutzer eine ungemein effektive und sichere Behandlungsvorrichtung zur Verfügung gestellt werden kann.

Durch eine Hintereinanderschaltung der Sicherheitsschranken kann überraschend gewährleistet werden, dass die Behandlungsfläche nicht in einen Temperaturbereich gelangt, welcher den Patienten gefährden könnte.

In einer bevorzugten Ausführungsform umfasst der hardwareimplementierte Temperaturwächter mindestens einen zweiten Temperatursensor zur Messung der Temperatur der Behandlungsfläche und einen Komparator, wobei der Komparator die Temperatur der Behandlungsfläche mit der Maximaltemperatur vergleicht und bei Überschreiten der Maximaltemperatur die Stromzufuhr zu dem Heizelement unterbindet.

Im Sinne der Erfindung bezeichnet ein Komparator bevorzugt eine elektronische Schaltung zum Vergleichen zweier Spannungen, wobei am Ausgang in binärer Weise angezeigt wird, welcher der beiden Spannungen höher ist. Im Stand der Technik sind hinreichend verschiedene Komparatoren bekannt, welche geeignet sind aus zwei analogen Spannungen ein binäres Ausgangsignal auszugeben, welche aussagt, welche der Eingangsspannung höher ist. Als Beispiel für eine Komparator-Schaltung sei der Schmitt-Trigger genannt. Es ist bevorzugt, dass an einem Eingang des Komparators mit Hilfe eines Spannungsverteilers ein Referenzwert für eine Spannung angelegt wird. Dieser Referenzwert entspricht bevorzugt dem Spannungswert, welcher der zweite Temperatursensor aufweisen würde, wenn die Temperatur der Behandlungsfläche gleich der Maximaltemperatur ist. An dem zweiten Eingang des Komparators liegt bevorzugt die Ausgangsspannung des Temperatursensors an, welche von der Temperatur der Behandlungsfläche abhängt. Ein besonders bevorzugter Temperatursensor umfasst zu diesem Zweck einen NTC-Thermistor, d.h. einen Heißleiter. Dieser besitzt einen negativen Temperaturkoeffizienten, sodass bei einer Erhöhung der Temperatur der Widerstand fällt und ein höherer Strom fließt. Es können aber auch Kaltleiter, d.h. PTC-Thermistoren verwandt werden, welche einen positiven Temperaturkoeffizienten besitzen, sodass bei einer Erhöhung der Temperatur der Widerstand steigt und ein niedrigerer Strom fließt.

Steigt die Temperatur der Behandlungsfläche, bewegt sich der durch den zweiten Temperatursensor gesteuerte Spannungswert am Komparator auf den Referenzwert der Spannung zu, welcher der Maximaltemperatur entspricht. Sobald die Temperatur die Maximaltemperatur überschreitet, ändert sich das Ausgangsignal am Komparator binär. Der Komparator ist bevorzugt in die Stromversorgung der Heizelemente integriert. D.h. bevor die Temperatur der Behandlungsfläche die Maximaltemperatur erreicht, gibt der Komparator bevorzugt die Versorgungsspannung der Heizelemente frei. Sobald jedoch die Temperatur höher als die Maximaltemperatur ist, schaltet der Ausgang des Komparators ab und unterbricht die Versorgungsspannung der Heizelemente. Fällt die Temperatur der Behandlungsfläche wieder, wird die Versorgungsspannung vorteilhafterweise wieder durch den Komparator freigegeben. Hierdurch kann ein reversibles An- und Abschalten der Heizelemente nur für den Zeitraum erfolgen, während die Temperatur der Behandlungsfläche die Maximaltemperatur überschreitet. Weiterhin kann es bevorzugt sein, dass der Komparator durch die Regelvorrichtung beim Starten der Vorrichtung freigeschaltet wird. Erfolgt somit kein ordnungsgemäßer Start der Vorrichtung ist der Komparator in der Voreinstellung so konfiguriert, dass die Spannungsversorgung der Heizelemente unterbrochen ist.

Die beschriebene bevorzugte Ausführungsform des hardwareimplementierten Temperaturwächters hat sich in Tests als besonders robust und zuverlässig erwiesen. Aufgrund der Reversibilität der Sicherheitsabschaltung und einfachen Konstruktionsweise zeichnet sich die bevorzugte Ausführungsform weiterhin durch niedrige Herstellungs- und Wartungskosten aus.

Durch die von der Regelvorrichtung unabhängige Bauweise samt eigenem Temperatursensor kann ein zuverlässiger Betrieb auch bei Ausfall eines Bauteils der Regelvorrichtung gewährleistet werden. Ebenso ist ein hardwareimplementierter Temperaturwächter in beschriebener Form unter Ausnutzung eines Komparators besonders schnell, da Komparatoren weit verbreitete elektronische Bauteile sind, die sich neben ihrer Zuverlässigkeit durch ihre schnelle Schaltfähigkeit ausweisen. So gibt es beispielsweise Komparatoren mit Schaltzeiten von Nanosekunden (ns) und darunter. Es konnte überraschenderweise festgestellt werden, dass durch den Einsatz von Komparatoren in der Schaltung aufgrund Ihrer Schnelligkeit ein besonders wirksamer Schutzmechanismus gegen Überhitzen der Behandlungsfläche aufgebaut werden konnte.

In einer bevorzugten Ausführungsform weist die Schmelzsicherung einen Schwellenwert für einen maximalen Strom auf, welcher der Erhitzung der Behandlungsfläche auf 65 °C für 1 Sekunde entspricht. Tests haben gezeigt, dass erst eine Temperaturerhöhung von über 65 °C für länger als 1 Sekunde sehr kritisch für das Schmerzempfinden ist und zu Schädigungen der Hautpartien führen kann. Vorteilhafterweise wird durch Einstellung der Schmelzsicherung auf diese Parameterwerte die Schmelzsicherung nicht vorzeitig bei unkritischen Temperaturerhöhungen der Behandlungsfläche ausgelöst. Hierdurch kann Wirtschaftlichkeit erhöht werden, ohne einen Kompromiss in Bezug auf die Sicherheit einzugehen. Der Fachmann weiß anhand der elektrischen Parameter der Heizelemente, welche Schmelzsicherung gewählt werden sollte, um die angebenden Werte zu gewährleisten. Hierzu kann auch eine Messung der Stromzufuhr bei gleichzeitiger Messung der Temperatur der Behandlungsfläche erfolgen. Weiterhin ist es besonders bevorzugt eine flinke Schmelzsicherung zu verwenden, welche auf eine Stromerhöhung innerhalb von bevorzugt weniger als 20 Millisekunden (ms) reagiert. So wurde erkannt, dass auch eine kurzfristige Stromerhöhung von weniger als 20 ms aufgrund der thermischen Trägheit der Behandlungsfläche zu einer Temperaturerhöhung von länger als 1 Sekunde führen kann.

Gegenüber nicht rückstellenden, rein temperaturabhängigen Thermosicherungen, welche ebenfalls durch Abschmelzen funktionieren, hat die hier verwendete stromabhängige Schmelzsicherung einige Vorteile. Bei nicht rückstellenden, reinen temperaturabhängigen Thermosicherungen geschieht das Abschmelzen nicht bei Anliegen eines Stromes oberhalb eines Schwellenwertes, sondern erst bei Anliegen einer externen Temperatur, welche größer ist als eine definierte Maximaltemperatur. Gegenüber nicht rückstellenden reinen temperaturabhängigen Thermosicherungen können stromabhängige Schmelzsicherungen somit schon reagieren, bevor in Folge eines länger wirkenden erhöhten Stroms eine bestimmte unerwünschte Temperatur überhaupt erreicht wird. Ebenso brauchen nicht rückstellende reine temperaturabhängige Thermosicherungen bei Anliegen einer externen Temperatur, welche größer ist als eine definierte Maximaltemperatur, immer eine gewisse Reaktionszeit. Somit kann es zu gefährlichen, weiteren Temperaturerhöhungen kommen. Stromabhängige Schmelzsicherungen reagieren im Gegensatz dazu schneller und mit minimalen systembedingten Latenzzeiten.

In einer bevorzugten Ausführungsform beträgt der Schwellenwert der Schmelzsicherung bevorzugt zwischen 1 A und 2,5 A besonders bevorzugt ungefähr 2 A. Tests haben gezeigt, dass in Bezug auf die bevorzugten Heizelemente die genannten Schwellenwerte besonders zuverlässig gewährleisten, dass die Temperatur der Behandlungsfläche eine Temperatur von 65 °C bis 70 °C für nicht länger als 1 Sekunde überschreitet. Durch das Schmelzen der Schmelzsicherung ab Stromwerten von 1 A bis 2.5 A kann somit sichergestellt werden, dass die Temperatur der Behandlungsfläche nicht in einen gesundheitsgefährdenden Bereich kommen kann. So kommt es bei einer regulären Behandlung zu einem regulären Behandlungsstrom, welcher unterhalb von 2,5 A, bevorzugt 1 A liegt. Tritt ein Fehler auf, z.B. bei einem Durchheizen, so fließt ein erhöhter Strom. In diese Falle Sicherung greift die Sicherung ein und verhindert wirksam ein unkontrolliertes Aufheizen.

Durch die vorteilhafte Auswahl der Maximaltemperatur des hardwareimplementierten Temperaturwächters auf einen Wert zwischen 54 °C und 58 °C bevorzugt von ungefähr 56 °C kann außerdem sichergestellt werden, dass der Abstand groß genug ist zur Temperatur beim Auslösen der Schmelzsicherung in Folge eines Stromwertes oberhalb des Schwellenwertes. So kann ein versehentliches Auslösen der Schmelzsicherung, welche mindestens einen Austausch der Sicherung zur Folge hätte, vermieden werden, so lange keine schwerwiegende Störung, welche den hardwareimplementierten Temperaturwächter einschließt, vorliegt.

In einer bevorzugten Ausführungsform der Erfindung ist die Vorrichtung dadurch gekennzeichnet, dass die Vorrichtung einen Datenspeicher zur Speicherung der Systemdaten und/oder Fehlermeldungen umfasst. Bevorzugt kann der Datenspeicher in der Regelvorrichtung integriert vorliegen. Zu den bevorzugten Systemdaten gehört ein Zähler für die Behandlungszyklen, welcher bevorzugt separat die Verwendung verschiedene Typen von Behandlungszyklen zählt. Kann beispielsweise ein kurzer oder ein langer Behandlungszyklus ausgewählt werden, so wird dieser separate gezählt. Weiterhin umfassen die Systemdaten bevorzugt einen Boot Zähler, also eine Zähler dafür wie oft die Vorrichtung gestartet wurde sowie eine Angabe der Fehlermeldungen mit aktuellem Fehlerstand.

Bevorzugt können folgende Fehlermeldungen gespeichert: Ein "Reset" zeigt an, dass der Spannungswächter einen Reset ausgelöst hat. Ein "Watchdog" zeigt an, dass in der Firmware ein Watchdog-Reset aufgetreten ist, d.h. ein Systemneustart aufgrund eines Softwarefehlers. Bevorzugt kann für die Fehlermeldung festgestellt werden in welchem Programmmodus sich das Gerät bei dem Auftreten des Fehlers befand. Ein "Temperatur zu hoch" kann anzeigen, dass die am Temperatursensor gemessene Temperatur zu hoch ist, oder dass der Temperatursensor defekt ist. Ein "Temperatur zu niedrig" kann anzeigen, dass die am Temperatursensor gemessene Temperatur zu niedrig ist, oder dass der Temperatursensor defekt ist. Ein "Behandlungstemperatur erreicht" kann anzeigen, ob die gewünschte Behandlungstemperatur erreicht wurde oder ein Fehler während der Vorheizphase aufgetreten ist.

Vorteilhafterweise können die gespeicherten Systemdaten und Fehlermeldungen für die Diagnose und Problembehandlung für die Vorrichtung eingesetzt werden.

So können diese Daten beispielsweise durch einen Datenaustausch mit dem Mobilgerät ausgelesen werden

Anhand der Daten ist es möglich den aufgetretenen Fehler, z.B. "Temperatur zu hoch", mit weiteren Systemdaten zur Anzahl der Behandlungszyklen oder Watchdog-Resets zu korrelieren. Anhand dieser Daten kann somit sowohl insbesondere auch nach einer Entwicklungsphase anhand der durch die Nutzer zusammengetragenen Daten, die Sicherheitsmerkmale der Vorrichtung kontinuierlich optimiert werden. Die Möglichkeit, dass die Vorrichtung eine Speicherung von Systemdaten und Fehlermeldungen umfasst, erlaubt somit die kontinuierliche Verbesserung der Hardware- sowie Softwarebestandteile der Vorrichtung anhand aussagekräftiger Daten.

In einer weiteren bevorzugten Ausführungsform ist die Vorrichtung dadurch gekennzeichnet, dass auf der Regelvorrichtung einer Firmware installiert vorliegt, welche mindestens die Temperaturregulation der Behandlungsfläche steuert, wobei die Firmware einen watchdog counter (WDC) umfasst, welcher überwacht, ob die Firmware ausgeführt wird. Im Sinne der Erfindung wird bevorzugt unter der Firmware eine Software, d.h. die Anleitung für ein computer-implementiertes Verfahren verstanden, welches in der Regelvorrichtung bevorzugt in dem Mikroprozessor eingebettet vorliegt. D.h. die Firmware umfasst bevorzugt jene Software, welche mit der Hardware der Vorrichtung, d.h. insbesondere mit den Heizelementen und Temperatursensoren funktional verbunden ist. Vorzugsweise wird die Firmware mit dem Start der Vorrichtung ausgeführt und übernimmt die Kontroll- und Steuerungsfunktion dieser Hardware-Komponenten der Vorrichtung. Somit wertet die Regelvorrichtung bevorzugt auf Basis der Firmware z.B. die Messdaten der Temperatursensoren sowie Nutzereingaben aus, um während des Behandlungszyklus die Stromzufuhr für die Heizelemente zu steuern. Im Sinne der Erfindung bezeichnen hardwareimplementierte Bauelemente bevorzugt Bauelemente, deren Funktion unabhängig von einer korrekten Ausführung der Firmware sichergestellt ist. Wie obig beschrieben ist der Temperaturwächter hardwareimplementiert, sodass dessen Funktion, d.h. eine Begrenzung der Maximaltemperatur, unabhängig von einer korrekten Ausführung der Firmware auf der Regelvorrichtung erfolgen kann. Selbst bei einem Systemabsturz der Firmware kann der hardwareimplementierte Temperaturwächter daher schnell und korrekt die Maximaltemperatur der Behandlungsfläche begrenzen.

In der besonders bevorzugten Ausführungsform wird die Firmware der Regelvorrichtung mit Hilfe eines hardwareimplementierten Watchdog Counters überwacht. Besonders bevorzugt handelt es sich dabei um einen Time-out-Watchdog. Bevorzugt wird der Time-Out-Watchdog vor dem Start der Behandlungsphase durch die Firmware aktiviert. Während der Behandlungsphase wird von der Firmware innerhalb eines vorbestimmten Zeitintervalls ein Signal an den Time-Out-Watchdog gesendet, um diesen zurückzusetzen. Falls der Time-Out-Watchdog nicht zurückgesetzt wird, führt dies bevorzugt zu einem Neustart der Firmware. Das Zeitintervall richtet sich bevorzugt an die Zeit, welche vorgesehen ist um eine Temperaturmessung und Regelung der Heizelemente durch die Firmware durchzuführen und kann z.B. zwischen 2 ms und 10 ms betragen. Durch einen solchen Time-Out-Watchdog kann vorteilhafterweise sichergestellt werden, dass mindestens während der Behandlungsphase der Vorrichtung die Firmware korrekt funktioniert und die Temperatur der Behandlungsfläche überwacht wird. Durch einen hardwareimplementierten Watchdog zur Überwachung der Firmware, bevorzugt zum Beispiel mit Hilfe eines Time-Out-Watchdogs, kann somit sichergestellt werden, dass falls die Firmware nicht korrekt funktioniert und das vorgegebene Zeitintervall nicht eingehalten wird, die Behandlungsphase abgebrochen wird. Somit ist ein weiteres Sicherheitsfeature der Vorrichtung zusätzlich zu den oben erwähnten vorhanden, welches insbesondere im Zusammenspiel mit dem hardwareimplementierten Temperaturwächter dafür sorgt, dass auch bei nicht korrekt funktionierender Firmware ein Überhitzen der Behandlungsfläche ausgeschlossen ist.

In einer bevorzugten Ausführungsform umfasst die Vorrichtung mindestens einen Kontaktsensor, welcher feststellen kann, ob die Behandlungsfläche in Kontakt mit einer Haut ist oder nicht.

Im Sinne der Erfindung bezeichnet der Kontaktsensor eine Einheit, welche anhand von Messdaten und deren Analyse eine Aussage darüber treffen kann, ob die Behandlungsfläche in Kontakt mit einer Haut, bspw. einer Lippe oder einer anderen Hautpartie, ist oder nicht. Vorzugsweise umfasst der Kontaktsensor hierzu einen Sensor oder eine Messeinheit, welche mit der Regelungsvorrichtung verbunden ist, wobei die Regelungsvorrichtung die Auswertung der Messdaten vornehmen kann.

Mittels des Kontaktsensors und der Information darüber zu welchem Zeitpunkt die Behandlungsfläche die Haut kontaktiert, kann eine besonders präzise Regulation des Wärmeflusses zur Behandlung von Juckreiz oder Herpes erzielt werden. Beispielsweise kann der Beginn der Aufheizphase davon abhängig gemacht werden, ob ein Kontakt mit der Haut vorliegt. Auch der Zeitraum der Behandlungsphase kann zuverlässig aufgezeichnet werden, um erfolgte Behandlungen zu überwachen und ggf. weitere Anwendungen daraufhin anzupassen. Auch hinsichtlich von Sicherheitsaspekten erlaubt der Kontaktsensor eine verbesserte Kontrolle. So kann mit Hilfe eines Kontaktsensors vermieden werden, dass sich die Behandlungsfläche ohne Wissen oder Wollen des Nutzers aufheizt.

In einer weiteren Ausführungsform der Erfindung ist die Vorrichtung dadurch gekennzeichnet, dass die Regelvorrichtung auf Basis einer Korrelation der Messdaten eines Temperatursensors und Daten über die Ansteuerung des Heizelementes feststellen kann, ob die Behandlungsfläche in Kontakt mit der Haut vorliegt.

In einer weiteren Ausführungsform wird der Kontaktsensor durch einen Temperatursensor und einer Regelvorrichtung zur Steuerung der Heizelemente gebildet. Grundlage der Kontaktmessung ist die Erkenntnis, dass der notwendige Stromfluss zum Erreichen oder Aufrechterhalten einer Temperatur davon abhängt, ob die Behandlungsfläche eine thermische Last (z.B. eine Haut) kontaktiert. Im Falle eines Aufheizens der Behandlungsfläche beim Hautkontakt erfolgt ein Wärmeübertrag, welcher durch eine erhöhte Energiezufuhr an die Heizelemente ausgeglichen werden muss. Durch Auswertung des Strom- und Temperaturverlaufes können zuverlässige Aussagen darüber getroffen werden, ob die Behandlungsfläche eine Haut kontaktiert. Bevorzugt kann der Regelungsvorrichtung zu diesem Zweck Referenzdaten bereitgestellt werden.

In einer bevorzugten Ausführungsform der Erfindung umfasst die Regelvorrichtung Referenzdaten über eine Korrelation der Temperatur der Behandlungsfläche mit der Ansteuerung des mindestens einen Heizelementes im Falle, dass die Behandlungsfläche in Kontakt mit der Haut oder mit Luft vorliegt. Die Referenzdaten können beispielsweise Verhältnisse aus gemessener Temperatur und dafür notwendiger Stromzufuhr umfassen. Besonders bevorzugt umfassen die Referenzdaten derartige Verhältnisse für einen Temperaturverlauf, sodass anhand der Messung des aktuellen Verhältnisses aus Temperatur und Stromzufuhr besonders präzise bestimmt werden kann, ob die Behandlungsfläche eine Haut kontaktiert. Vorteilhafterweise kann mithilfe einer derartigen Regulation nicht nur ein Kontakt mit der Haut im Vergleich zur Luft, sondern ebenfalls ein Kontakt mit der Haut im Vergleich zu Materialien mit anderen thermischen Eigenschaften zuverlässig unterschieden werden.

In einer weiteren bevorzugten Ausführungsform können die Referenzdaten die durchschnittlich an der Haut oder der Luft abgegebene Wärmemenge umfassen. Dabei können die Referenzdaten Korrelationen zwischen Behandlungstemperatur und abgegebener Wärme enthalten.

Mittels eines derartigen Kontaktsensors kann besonders präzise eine Behandlungstemperatur eingestellt werden, welche einer durchschnittlichen Temperatur der Außenseite der Behandlungsfläche entspricht, während diese eine Haut kontaktiert.

In einer weiteren bevorzugten Ausführungsform der Erfindung ist die Vorrichtung dadurch gekennzeichnet, dass die Behandlungsfläche eine Dicke zwischen 0,2 mm und 5 mm, bevorzugt zwischen 0,5 mm und 2 mm, besonders bevorzugt zwischen 1 mm und 1,5 mm aufweist und aus einem Material besteht, welches eine Wärmeleitfähigkeit bei 50°C zwischen 20 W/mK und 400 W/mK, bevorzugt zwischen 100 und 350 W/mK aufweist. Die Wärmeleitfähigkeit (auch als Wärmeleitzahl bezeichnet) charakterisiert bevorzugt die thermischen Eigenschaften des Materials, aus welchem die Behandlungsfläche gefertigt ist. Die Wärmeleitfähigkeit gibt an, wie hoch die Wärmemenge ist, welche durch die Behandlungsfläche geleitet wird, wenn an dieser ein Temperaturgradient anliegt. Neben der Wärmeleitfähigkeit hängt der Wärmetransport von der Dicke der Behandlungsfläche, der Größe der Behandlungsfläche und dem Temperaturunterschied zwischen der Innenseite der Behandlungsfläche (Kontakt mit den Heizelementen) und der äußeren Seite der Behandlungsfläche (Kontakt mit der Haut) ab. Die Wärmeleitfähigkeit wird bevorzugt als Verhältnis der transportierten Wärmeleistung Watt (W) pro Temperaturdifferenz in Kelvin (K) und pro Meter (m) angegeben. Die Wärmeleitfähigkeit kann aber auch bevorzugt als Verhältnis der transportierten Wärmeleistung Watt (W) pro Temperaturdifferenz in Millikelvin (mK) angegeben werden. Da die Wärmeleitfähigkeit sich weiterhin in Abhängigkeit der Temperatur leicht ändern kann, wird vorliegend die Referenztemperatur mit 50 °C angegeben. Die Dicke der Behandlungsfläche bezeichnet weiterhin bevorzugt die Ausdehnung der Behandlungsfläche zwischen der äußersten Fläche, welche die Haut kontaktiert und der innersten Fläche, an welcher die Heizelemente anliegen.

Bei einer Dicke der Behandlungsfläche zwischen 0,2 mm und 5 mm, bevorzugt zwischen 0,5 mm und 2 mm und besonders bevorzugt zwischen 1 mm und 1,5 mm ergibt sich in Kombination mit der bevorzugten Wärmeleitfähigkeit zwischen 100 und 350 W/mK eine therapeutisch besonders wirkungsvolle Wärmeabgabe an die Haut. In experimentellen Versuchen haben sich die bevorzugt genannten Parameter als überraschend vorteilhaft erwiesen. So vermeidet eine derart konzipierte Behandlungsfläche eine zu schnelle Abgabe der Wärme an die betreffenden Hautpartien, wodurch ein unangenehm stechender Schmerz ausgelöst werden kann. Dennoch erfolgt die Wärmeabgabe in einem Zeitraum, welcher ausreichend impulshaft erfolgt, um effektiv die Rezeptoren anzuregen und einen Juckreiz zu überlagern. Die genannten Parameter stellen daher eine optimierte Auswahl dar, welche für einen Fachmann nicht nahelag. Außerdem wird durch die Parameter bevorzugt gewährleistest, dass die Wärme der Behandlungsfläche während der Behandlungsphase schnell und effektiv an die Hautpartie abgegeben wird, sodass ein Auftreten von Restwärme keine Gefährdung darstellt.

In einer bevorzugten Ausführungsform umfasst die Behandlungsfläche Keramik oder Gold. Es ist besonders bevorzugt, dass die Behandlungsfläche aus Gold oder Keramik besteht. Die Materialien Keramik und Gold fallen zum einen in den experimentell ermittelten, bevorzugten Bereiche der Wärmeleitfähigkeit. Weiterhin speichern die Materialien bevorzugt selbst nicht zulange Wärme, sodass diese Materialien sich relative schnell aufheizen und wieder abkühlen. Dies erlaubt eine erhöhte Sicherheit, da nach der Behandlungsphase sichergestellt werden kann, dass die Behandlungsfläche aufgrund von Restwärme keine Gefährdung darstellt. Darüber hinaus zeichnen sich sowohl Keramik als auch Gold durch eine hohe biologische Verträglichkeit bei den bevorzugten Behandlungstemperaturen aus. Allergische Reaktion oder anderen unerwünschte Nebenwirkungen können bei dieser Wahl der Materialien besonders effektiv vermieden werden.

In einer bevorzugten Ausführungsform erfolgt eine direkte Bestückung der Behandlungsfläche mit den Heizelementen, um einen besseren Transfer der Wärme zu gewährleisten. Der Fachmann kennt geeignete Technologien zur direkten Bestückung von beispielsweise Keramik, bzw. Keramik-Leiterplatten.

In einer bevorzugten Ausführungsform der Erfindung ist die Vorrichtung dadurch gekennzeichnet, dass die Behandlungsfläche durch eine Markierung umrandet ist, welcher in Abhängigkeit des Behandlungszyklus leuchtet. Als Markierung kann es zum Beispiel bevorzugt sein die Behandlungsfläche mit einem Lichtleiter zu umranden. Dieser kann beispielsweise während der Aufheizphase oder aber während der Behandlungsphase zum Leuchten gebracht werden. Es hat sich gezeigt, dass durch eine explizite leuchtende Anzeige der Position der Behandlungsfläche der Erfolg der hyperthermischen Behandlung erhöht werden kann. So wird durch die visuelle Markierung ein zentriertes Auflegen auf den betroffenen Hautpartien gefördert, sodass der Wärmeimpuls zielgerichtet auf diese Hautpartien aufgebracht werden kann. Durch die beleuchtete Markierung kann eine Benutzung auch im Dunkeln, beispielsweise in einem Zelt im Freien bei Nacht, unproblematisch vorgenommen werden kann.

In einer weiteren bevorzugten Ausführungsform betrifft die Erfindung einen Kit umfassend
a) eine erfindungsgemäße Vorrichtung oder eine bevorzugte Ausführungsform und
b) eine Software zur Installation auf einem Mobilgerät
wobei die Software konfiguriert ist über das Interface der Vorrichtung eines von mindestens zwei Behandlungsprogrammen auszuwählen.

In einer weiteren bevorzugten Ausführungsform betrifft die Erfindung ein System umfassend
a) eine erfindungsgemäße Vorrichtung oder eine bevorzugte Ausführungsform und
b) ein Mobilgerät
wobei auf dem Mobilgerät eine Software installiert vorliegt, welche konfiguriert ist über das Interface der Vorrichtung eines von mindestens zwei Behandlungsprogrammen auszuwählen.

Der durchschnittliche Fachmann erkennt, dass technische Merkmale und Vorteile bevorzugter Ausführungsformen der erfindungsgemäßen Vorrichtung auch für den erfindungsgemäßen Kit und das System gelten.

Unter der Software wird bevorzugt ein Anwendungsprogramm (,App') verstanden, welchs auf einem Mobilgerät, wie beispielsweise einem Smartphone installiert werden kann, um eine Verbindung mit der Vorrichtung aufzunehmen und verschiedene Funktionen durchzuführen. Bevorzugte wird somit die Interaktion zwischen Mobilgerät und der Vorrichtung mittels der Software durchgeführt. Insbesondere ist die Software dafür konfiguriert über das Interface der Vorrichtung eines von mindestens zwei Behandlungsprogrammen auszuwählen. Zu diesem Zweck kann die Software dafür konfiguriert sein an einem Touchdisplay des Mobilgerätes die möglichen Behandlungsprogramme anzuzeigen und zur Auswahl zu stellen. Der Fachmann kennt selbstverständlich verschiedene Möglichkeiten, um eine anschauliche und intuitive Bedienung zu gewährleisten.

Bevorzugte Ausführungsformen, welche im Zusammenhang mit der Vorrichtung insbesondere mit deren Interaktion mit einem Mobilgerät offenbart wurden, gelten bevorzugt gleichermaßen für den Kit und die umfasste Software. Der Fachmann erkennt, dass die beschriebenen Interaktionsmöglichkeiten eines Mobilgerätes mit der Vorrichtung bevorzugt durch die Software auf dem Mobilgerät bereitgestellt werden kann. Beispielweise wurde in einer bevorzugten Ausführungsform ein Datenaustausch zwischen dem Mobilgerät und der Vorrichtung offenbart und Vorteile einer Auswertung der übersandten Daten sowie ggf. Vernetzung mit anderen Benutzerdaten genannt. Der Fachmann erkennt, dass es zu diesem Zweck bevorzugt eine entsprechende Software konfiguriert wird und kann die konkrete Ausgestaltung der Software routinemäßig vornehmen.

## Patentansprüche

1. Vorrichtung zur hyperthermischen Behandlung von Juckreiz oder Herpeserkrankungen umfassend
a. mindestens eine Behandlungsfläche
b. ein Bedienelement und
c. eine Regelvorrichtung, welche konfiguriert ist nach Betätigung des Bedienelementes durch Erhitzen mindestens eines Heizelements gemäß vorgegebener Behandlungsparameter eines Behandlungsprogrammes die Behandlungsfläche in einer Aufheizphase auf eine Behandlungstemperatur zwischen 40° - 65°C zu regulieren und für eine Behandlungsdauer zwischen 1 und 12 Sekunden zu halten
**dadurch gekennzeichnet, dass**
auf der Regelvorrichtung mindestens zwei unterschiedliche Behandlungsprogramme gespeichert vorliegen, welche unterschiedliche Behandlungsparameter festlegen und wobei die Vorrichtung mindestens ein Interface zur Verbindung mit einem Mobilgerät aufweist und die Vorrichtung konfiguriert ist für die Auswahl eines der mindestens zwei Behandlungsprogramme durch das Mobilgerät, sodass nach Betätigung des Bedienelement das ausgewählte Behandlungsprogramm ausgeführt wird.

2. Vorrichtung nach Anspruch 1
**dadurch gekennzeichnet, dass**
die Vorrichtung derart konfiguriert ist, dass eine Änderung der Behandlungsparameter, welche durch die Behandlungsprogramme festgelegt werden, durch das Mobilgerät ausgeschlossen ist.

3. Vorrichtung nach einem oder mehreren der vorigen Ansprüche,
**dadurch gekennzeichnet, dass**
die durch die Behandlungsprogramme festgelegten Behandlungsparameter die Behandlungstemperatur und die Behandlungsdauer umfassen, wobei bevorzugt zusätzlich eine Dauer für die Aufheizphase als Behandlungsparameter festgelegt wird und/oder auf der Regelvorrichtung zwischen 2 und 10, bevorzugt zwischen 4 und 8 Behandlungsprogramme gespeichert vorliegen.

4. Vorrichtung nach einem oder mehreren der vorigen Ansprüche,
**dadurch gekennzeichnet, dass**
die Vorrichtung nur genau einen Auslöser für das Erhitzen der Behandlungsfläche gemäß des ausgewählten Behandlungsprogrammes aufweist und an der Vorrichtung selbst manuell keine Auswahl des Behandlungsprogrammes erfolgen kann.

5. Vorrichtung nach einem oder mehreren der vorigen Ansprüche,
**dadurch gekennzeichnet, dass**
die Vorrichtung derart konfiguriert ist, dass unabhängig von einer bestehenden Kommunikation zu einem Mobilgerät bei Betätigung ein zuletzt ausgewähltes Behandlungsprogramm ausgeführt wird.

6. Vorrichtung nach einem oder mehreren der vorigen Ansprüche,
**dadurch gekennzeichnet, dass**
das Interface und die Regelvorrichtung so konfiguriert sind, dass nur bei erfolgreicher Authentifizierung eine Auswahl eines der mindestens zwei Behandlungsprogramme mittels eines Mobilgerätes ermöglicht wird.

7. Vorrichtung nach einem oder mehreren der vorigen Ansprüche,
**dadurch gekennzeichnet, dass**
die in den mindesten zwei Behandlungsprogrammen festgelegten Behandlungstemperaturen zwischen 40 °C bis 60 °C, bevorzugt zwischen 45°C und 53°C, besonders bevorzugt zwischen 47°C und 53°C betragen.

8. Vorrichtung nach einem oder mehreren der vorigen Ansprüche,
**dadurch gekennzeichnet, dass**
in einem ersten der auswählbaren Behandlungsprogramme einer Behandlungsdauer von 1 - 3 Sekunden, während in einem zweiten der auswählbaren Behandlungsprogramme einer Behandlungsdauer zwischen 3-12 Sekunden festgelegt ist und/oder in einem ersten der auswählbaren Behandlungsprogramme einer Behandlungstemperatur zwischen 48°C und 53°C festgelegt ist, während in einem zweiten der auswählbaren Behandlungsprogramme eine Behandlungstemperatur zwischen 45°C und 48°C festgelegt ist.

9. Vorrichtung nach einem oder mehreren der vorigen Ansprüche,
**dadurch gekennzeichnet, dass**
die Größe der Behandlungsfläche weniger als 1 cm², bevorzugt zwischen 20 und 80 mm² beträgt und/oder
die Größe der Behandlungsfläche zwischen 1 cm² und 18 cm², bevorzugt zwischen 6 und 9 cm² beträgt.

10. Vorrichtung nach einem oder mehreren der vorigen Ansprüche,
**dadurch gekennzeichnet, dass**
die Vorrichtung einen Energiespeicher, bevorzugt ein Akku, besonders bevorzugt ein Lithium-Polymer und/oder ein Metall-hydrid-Akku umfasst und/ der Akku ein Festkörperakku, bevorzugt ein Lithium - Keramik-Akku ist.

11. Vorrichtung nach einem oder mehreren der vorigen Ansprüche,
**dadurch gekennzeichnet, dass**
das Interface zusätzlich zur Stromversorgung durch ein Mobilgerät konfiguriert ist und/oder das Interface zusätzlich für einen Datenaustausch mit einem Mobilgerät konfiguriert ist.

12. Vorrichtung gemäß einem oder mehreren der vorigen Ansprüche
**dadurch gekennzeichnet, dass**
die Vorrichtung mindestens einen ersten Temperatursensor zur Messung der Temperatur der Behandlungsfläche umfasst und die Regelvorrichtung basierend auf den Messdaten des Temperatursensors die Temperatur der Behandlungsfläche einstellt,
und/oder ein hardwareimplementierter Temperaturwächter die Maximaltemperatur der Behandlungsfläche reversibel begrenzt und eine Schmelzsicherung bei Kurzschluss oder ungeregeltem Durchheizen die Vorrichtung abschaltet.

13. Vorrichtung nach einem oder mehreren der vorigen Ansprüche,
**dadurch gekennzeichnet, dass**
die Vorrichtung einen Kontaktsensor umfasst, welcher feststellen kann, ob die Behandlungsfläche in Kontakt mit einer Haut ist oder nicht.

14. Kit umfassend
a) eine Vorrichtung gemäß einem oder mehreren der vorherigen Ansprüche und
b) eine Software zur Installation auf einem Mobilgerät
wobei die Software konfiguriert ist über das Interface der Vorrichtung eines von mindestens zwei Behandlungsprogrammen auszuwählen.

15. System umfassend
a) eine Vorrichtung gemäß einem oder mehreren der vorherigen Ansprüche und
b) ein Mobilgerät
wobei auf dem Mobilgerät eine Software installiert vorliegt, welche konfiguriert ist über das Interface der Vorrichtung eines von mindestens zwei Behandlungsprogrammen auszuwählen.
